(19)

Europäisches Patentamt
European Patent Office
Office européen des brevets

(11) **EP 3 858 336 A1**

(12) **EUROPEAN PATENT APPLICATION**

(43) Date of publication:
**04.08.2021 Bulletin 2021/31**

(51) Int Cl.:
***A61K 9/16*** (2006.01)

(21) Application number: **20305089.3**

(22) Date of filing: **31.01.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **ABIVAX**
  **75008 Paris (FR)**

• **CENTRE NATIONAL DE LA RECHERCHE SCIENTIFIQUE**
  **75794 Paris Cedex 16 (FR)**
• **UNIVERSITE DE MONTPELLIER**
  **34090 Montpellier (FR)**
• **Institut Curie**
  **75248 Paris Cedex 05 (FR)**

(72) Inventor: **The designation of the inventor has not yet been filed**

(74) Representative: **Nony**
  **11 rue Saint-Georges**
  **75009 Paris (FR)**

(54) **AMORPHOUS SOLID DISPERSION OF 8-CHLORO-N-(4-(TRIFLUOROMETHOXY)PHENYL)QUINOLIN-2-AMINE**

(57)  The present invention relates to an amorphous solid dispersion comprising ABX464 and at least one pharmaceutically acceptable carrier. The present invention also concerns a pharmaceutical composition comprising said ASD, processes for their preparation, their use as a medicament and more particularly their use in the treatment and/ or prevention of inflammatory diseases, diseases caused by viruses and/or cancer or dysplasia.

EP 3 858 336 A1

**Description**

**FIELD OF THE INVENTION**

The present invention relates to the field of pharmaceutical industry and concerns an amorphous solid dispersion (also named ASD in the present text) comprising 8-Chloro-N-(4-(trifluoromethoxy)phenyl)quinolin-2-amine (also named (8-chloroquinoline-2-yl)-(4-trifluoromethoxy-phenyl)-amine or ABX464), a pharmaceutical composition comprising said ASD, processes for their preparation, their use as a medicament and more particularly their use in the treatment and / or prevention of inflammatory diseases such as Inflammatory Bowel Disease, Rheumatoid Arthritis, pulmonary arterial hypertension, NASH (nonalcoholic steatohepatitis) and Multiple Sclerosis, diseases caused by viruses and/or cancer or dysplasia.

**BACKGROUND OF THE INVENTION**

WO2010/143169 application describes the preparation and use of compounds, and in particular quinoline derivatives including ABX464 or pharmaceutically acceptable salts thereof. ABX464 is currently under clinical development.

The inventors have stated that ABX464 is naturally highly crystalliferous and thus is spontaneously present under a specific unique stable and crystalline form.

In the pharmaceutical field, the formulations in which the active ingredient is under crystalline form are generally the first-intention formulations because of the general physical stability, API (active pharmaceutical ingredient) chemistry, low hygroscopicity, simpler control tests, robustness and ease of implementation of the formulations, purification step).

The implementation of ABX 464 in ASD formulations provides a new opportunity to improve the performance of a pharmaceutical product.

**SUMMARY OF THE INVENTION**

The present invention is intended to provide an ASD comprising 8-Chloro-N-(4-(trifluoromethoxy)phenyl)quinolin-2-amine as well as a pharmaceutical composition comprising said ASD which can be used both as a medicament and more particularly for treating and / or preventing inflammatory diseases such as Inflammatory Bowel Disease, Rheumatoid Arthritis, pulmonary arterial hypertension, NASH and Multiple Sclerosis, diseases caused by viruses and/or cancer or dysplasia.

The ASD technique allows to maintain ABX464 under a stable amorphous form during storage up to 100°C, in particular up to 80°C, in particular at least two weeks, and during administration of the pharmaceutical product in a subject in need thereof as shown in the experimental part (XRPD (X-Ray powder diffraction), mDSC (modulated differential scanning calorimetry) and TGA (Thermogravimetric analysis) characterizations). It has also been proved in the experimental part by using Fasted and Fed Human in vitro models that the amorphous form of ABX464 in the ASD can be kept after the administration of the product in a subject in need (patient) thereof and that the ASD in accordance with the present invention presents a significantly more important solubility compared to ABX464 in its unique crystalline form. In addition, the inventors have shown that the load of ABX464 in the ASD, more particularly after a spray-drying step (UV-HPLC (ultraviolet - High performance liquid chromatography) characterization) is maintained as wished.

The present invention thus provides an ASD comprising, or even consisting in, 8-chloro-N-(4-(trifluoromethoxy)phenyl)quinolin-2-amine or a pharmaceutically acceptable salt thereof and at least one pharmaceutically acceptable carrier.

Herein provided are also:

- a pharmaceutical composition comprising the ASD as defined in the present invention,
- processes for the preparation of the ASD and of the pharmaceutical composition as defined in the present invention,
- the ASD and the pharmaceutical composition as defined in the present invention for use as medicaments,
- the ASD and the pharmaceutical composition as defined in the present invention for use for treating and / or preventing inflammatory diseases such as Inflammatory Bowel Disease, Rheumatoid Arthritis, pulmonary arterial hypertension, NASH and Multiple Sclerosis, diseases caused by viruses and/or cancer or dysplasia.

As used herein, the term "pharmaceutically acceptable" refers to those compounds, materials, excipients, carrier, adjuvant, vehicle, compositions or dosage forms which are, within the scope of sound medical judgment, suitable for contact with the tissues of human beings and animals without excessive toxicity, irritation, allergic response or other problem complications commensurate with a reasonable benefit/risk ratio.

In the context of the invention, the term "treating" or "treatment", as used herein, means reversing, alleviating, inhibiting the progress of, or preventing inflammatory diseases such as Inflammatory Bowel Disease, Rheumatoid Arthritis, pulmonary arterial hypertension, NASH and Multiple Sclerosis, diseases caused by viruses and/or cancer or dysplasia.

The term "preventing", as used herein, means reducing the risk of onset or slowing the occurrence of a given phenomenon, namely in the present invention, inflammatory diseases such as Inflammatory Bowel Disease, Rheumatoid Arthritis, pulmonary arterial hypertension, NASH and Multiple Sclerosis, diseases caused by viruses and/or cancer or dysplasia. As used herein, « *preventing* » also encompasses « *reducing the likelihood of occurrence* » or « *reducing the likelihood of reoccurrence* ».

As used herein, the term "ambient temperature" or "room temperature" refers to a temperature ranging from 15°C to 30°C, more particularly from 18°C to 25°C.

## BRIEF DESCRIPTION OF THE FIGURES

**Figure 1a** represents a SEM image of ABX464 at 100x magnification (see example 2.3).

**Figure 1b** represents a SEM image of ABX464 at 1000x magnification (see example 2.3).

**Figure 2a** represents a SEM micrograph of ABX464:VA64 SDD at 10kx magnification after preparation through spray-drying (t=0h) (see example 2.3).

**Figure 2b** represents a SEM micrograph of ABX464:VA64 SDD at 1.5kx magnification after preparation through spray-drying (t=0h) (see example 2.3).

**Figure 2c** represents a SEM micrograph of ABX464:K30 SDD at 10kx magnification after preparation through spray-drying (t=0h) (see example 2.3).

**Figure 2d** represents a SEM micrograph of ABX464:K30 SDD at 1.5kx magnification after preparation through spray-drying (t=0h) (see example 2.3).

**Figure 3** represents XRPD diffractograms of two different ASDs which are ABX464:VA64 (diffractogram in the middle) and ABX464:K30 (diffractogram at the top) and of ABX 464 in its unique crystalline form (diffractogram at the bottom) (see example 2.2).

**Figure 4** represents a reversible M-DSC pattern recorded for 35/65 w/w (at 35% by weight of ABX464 drug load) ASD with PVP K30 (see example 2.2).

**Figure 5** represents a reversible M-DSC pattern recorded for 35/65 w/w (at 35% by weight of ABX464 drug load) ASD with PVP - VA64 (see example 2.2).

**Figure 6** represents TGA thermograms showing the % weight loss obtained for the ABX464:VA64 ASD (middle line, with circles), ABX464:K30 SDD (bottom line, with lozenges) and for the ABX464 in its unique crystalline form (top line, with crosses) (see example 2.4).

**Figure 7** represents a diagram illustrating a two - step dissolution / precipitation Fasted human "in vitro" model for ABX464:VA64 ASD (top line, with squares) and ABX464 in its unique crystalline form (bottom line, with triangles) (see example 3).

**Figure 8** represents a diagram illustrating a two - step dissolution / precipitation Fed human "in vitro" model for ABX464:VA64 ASD (top line, with squares) and ABX464 in its unique crystalline form (bottom line, with triangles) (see example 3).

**Figure 9** represents XRPD diffractograms, in simulated intestinal compartment, of NaCl (top line, that is to say first line), of ABX464 : VA64 ASD in suspension at the end of Fasted Human in-vitro model, that is to say Fassif, (second line), of ABX464 : VA64 ASD in suspension at the end of Fed Human in-vitro model, that is to say Fessif, (third line), and of ABX464 in its unique crystalline form (bottom line, that is to say fourth line) (see example 3).

**Figure 10** represents XRPD diffractograms of ABX 464 in its unique crystalline form (bottom line) and of ABX464:VA64 ASD which has been submitted to two different stress conditions (respectively at a temperature of 25°C and 60% relative humidity (middle line) ; and at a temperature of 40°C and 75% relative humidity (top line)). These diffractograms have been carried out after two weeks under these stress conditions. (see example 4).

**Figure 11** represents XRPD diffractograms of ABX 464 in its unique crystalline form (bottom line) and of ABX464:K30 ASD which has been submitted to two different stress conditions (respectively at a temperature of 25°C and 60% relative humidity (middle line) ; and at a temperature of 40°C and 75% relative humidity (top line)). These diffractograms have been carried out after two weeks under these stress conditions. (see example 4).

## DETAILED DESCRIPTION OF THE INVENTION

## AMORPHOUS SOLID DISPERSION ACCORDING TO THE INVENTION

As mentioned above, herein is provided an ASD comprising 8-chloro-N-(4-(trifluoromethoxy)phenyl)quinolin-2-amine or a pharmaceutically acceptable salt thereof and at least one pharmaceutically acceptable carrier.

In the context of the present invention:

- "ASD" means a glass solution, that is to say that ABX464 (the active pharmaceutical ingredient or API) is under an amorphous form. The pharmaceutically acceptable carrier is also under an amorphous form in which the ABX464 molecules (the solute molecules) are dispersed molecularly. A glass solution forms therefore homogeneous one-phase systems. ASD in the meaning of the present invention encompasses three categories depending on the presence of two or only one of the two following aspects: intermolecular interactions and molecular mobility. These three types are called co-amorphous ASD, amorphous solid solution, and stabilized ASD.
- "co-amorphous ASD" is an ASD having strong intermolecular interactions between the components forming this ASD but a high molecular mobility.
- "Amorphous solid solution" is an ASD having low molecular mobility but very weak intermolecular interactions between the components forming this ASD.
- "stabilized ASD" is an ASD having low molecular mobility and strong intermolecular interactions between the components forming this ASD.
- "amorphous" refers to a solid compound or a mixture of solid compounds that is/are not crystalline. An amorphous compound or a mixture of amorphous compounds possess(es) no long-range order but only display(s) short range order and hence do(es) not display a definitive X-ray diffraction pattern with reflections but only result in broad scattering.
- "ABX464: VA64" is the abbreviation for an ASD comprising ABX464 and polyvinylpyrrolidone - polyvinyl acetate copolymer as the pharmaceutical acceptable carrier.
- "ABX464: K30" is the abbreviation for an ASD comprising ABX464 and polyvinylpyrrolidone as the pharmaceutical acceptable carrier.

The ASD in accordance with the present invention thus encompasses the three above-mentioned types.

According to one embodiment, the present invention relates to a co-amorphous ASD comprising, or even consisting in, 8-chloro-N-(4-(trifluoromethoxy)phenyl)quinolin-2-amine or a pharmaceutically acceptable salt thereof and at least one pharmaceutically acceptable carrier.

According to another embodiment, the present invention relates to an amorphous solid solution comprising, or even consisting in, 8-chloro-N-(4-(trifluoromethoxy)phenyl)quinolin-2-amine or a pharmaceutically acceptable salt thereof and at least one pharmaceutically acceptable carrier.

According to another embodiment, the present invention relates to a stabilized ASD comprising, or even consisting in, 8-chloro-N-(4-(trifluoromethoxy)phenyl)quinolin-2-amine or a pharmaceutically acceptable salt thereof and at least one pharmaceutically acceptable carrier.

As it will be detailed below, the nature of the pharmaceutically acceptable carrier may play a role in the nature of the obtained ASD (co-amorphous ASD, stabilized ASD or amorphous solid solution).

According to a particular embodiment, the present invention relates to an amorphous solid dispersion consisting in 8-chloro-N-(4-(trifluoromethoxy)phenyl)quinolin-2-amine or a pharmaceutically acceptable salt thereof and at least one pharmaceutically acceptable carrier.

According to another particular embodiment, the present invention relates to an amorphous solid dispersion comprising, or even consisting in, 8-chloro-N-(4-(trifluoromethoxy)phenyl)quinolin-2-amine or a pharmaceutically acceptable salt thereof and at least one pharmaceutically acceptable polymer.

## 8-CHLORO-N-(TRIFLUOROMETHOXY)PHENYL)QUINOLIN-2-AMINE AND SALTS THEREOF

As mentioned-above, the ASD according to the invention comprises ABX 464 or a pharmaceutically acceptable salt thereof.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge et al., describe pharmaceutically acceptable salts in detail in J. Pharmaceutical Sciences, 1977, 66, 1-19, incorporated herein by reference. Pharmaceutically acceptable salts of the compounds of this invention include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, pivalate, propionate, stearate, succinate, sulfate, tartrate, thiocyanate, p-toluenesulfonate, undecanoate, valerate salts, and the like.

ABX 464 and salts thereof can be prepared by any process known by the skilled person, such as the one disclosed in WO2010/143169.

ABX 464 (when not comprised in an ASD according to the invention) is under a unique crystalline form which has a melting point of 120.5°C (±2°C) and shows the following main peaks expressed as degree 2-Theta angles by a XRPD analysis: 7.3, 14.6, 23.5, and 28.4 (each time ±0.2) and may further show the following additional peaks expressed as degree 2-Theta angles: 12.1, 17.3, 18.4, 23.0; 24.2, 24.9, 27.4 and 29.1 (each time ±0.2) and even optionally further the following additional peaks expressed as degree 2-Theta angles: 13.7, 16.3, 16.9, 18.1, 22.4, and 29.6 (each time ±0.2).

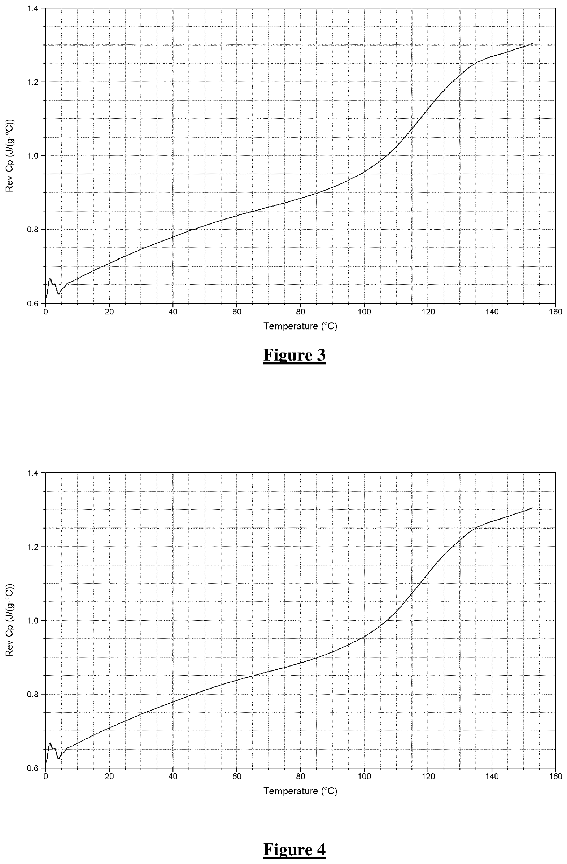

A characteristic X-ray powder diffractogram of this unique crystalline form of 8-Chloro-N-(4-(trifluoromethoxy)phenyl)quinolin-2-amine which was gently milled can be given in figure 3.

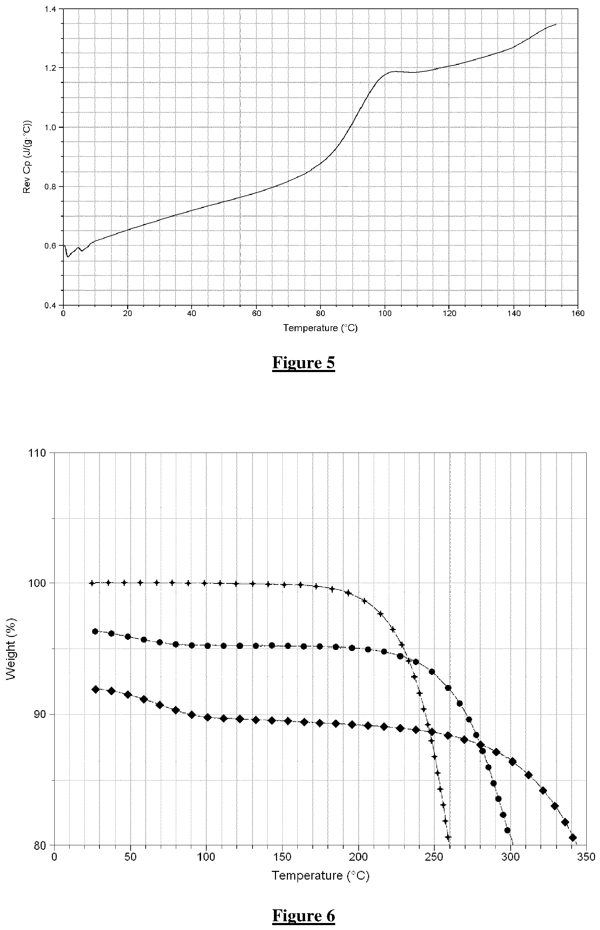

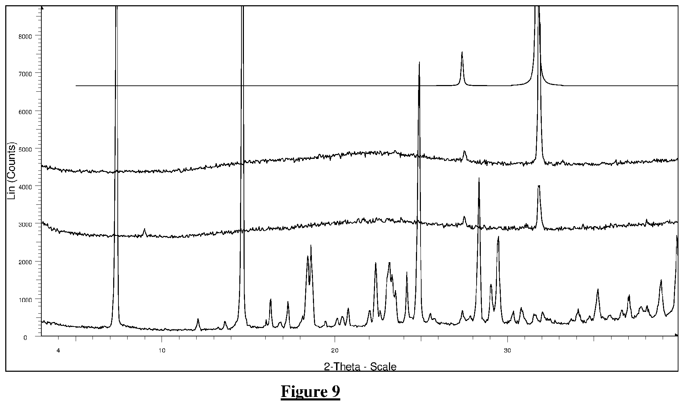

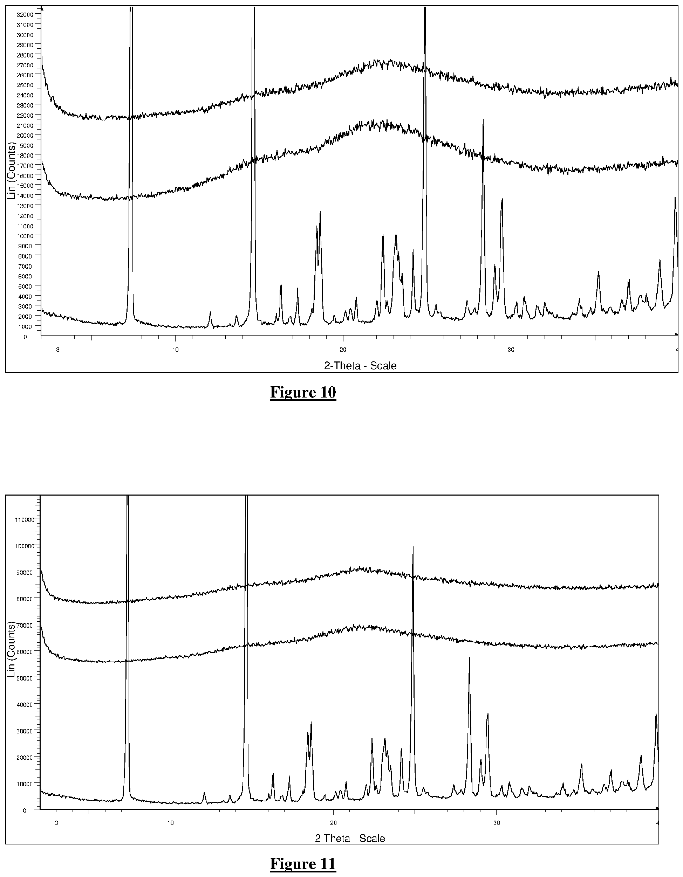

On the contrary, when ABX 464 is comprised in an ASD according to the invention, it is under an amorphous form as detailed below. This is demonstrated in the experimental part thanks to mDSC (figures 4 and 5, and example 2.2) and XRPD characterizations (figure 3, and example 2.2), even under specific stress conditions after two weeks (see figures 10 and 11, and example 4), and even in Fassif and Fessif *in vitro* models (see figure 9, and example 3) which simulate respectively fasted and fed state gastrointestinal fluids.

## PHARMACEUTICALLY ACCEPTABLE CARRIER

As mentioned above, the ASD according to the invention comprises at least one pharmaceutically acceptable carrier.

In one embodiment, the pharmaceutically acceptable carrier is selected from a polymer, a sugar, an acid, a surfactant, a cyclodextrin, pentaerythritol, pentaerythrityl tetraacetate, urea, urethane, hydroxy alkyl xanthins and mixtures thereof.

Among the sugars suitable for use in a solid amorphous dispersion of the invention can be cited dextrose, sucrose, galactose, sorbitol, maltose, xylitol, mannitol, lactose, and mixtures thereof.

Among the surfactants suitable for use in an amorphous solid dispersion of the invention can be cited polyoxyethylene stearate, poloxamer 188 (Poly(ethylene glycol)-*block*-poly(propylene glycol)-*block*-poly(ethylene glycol)), deoxycholic acid, tweens, spans, solutol (Macrogol-15 hydroxystearate), sodium lauryl sulfate, vitamin E, lauryl sulfate, and mixtures thereof.

Among the acids suitable for use in an amorphous solid dispersion of the invention can be cited carboxylic acids or other acidic compounds generally used to form pharmaceutically acceptable salts such as citric acid, succinic acid, malic acid, fumaric acid, tartaric acid and mixtures thereof.

Among the cyclodextrins suitable for use in an amorphous solid dispersion of the invention can be cited alpha-cyclodextrin, beta-cyclodextrin, gamma-cyclodextrin (that is to say each cyclodextrins which are not chemically modified), polymers of cyclodextrins (that is to say chemically modified cyclodextrins), and mixtures thereof.

The polymers suitable for use in an amorphous solid dispersion of the invention may have a Tg of at least 50°C, particularly of at least 80°C, and more particularly of at least 100°C.

In one embodiment, the polymers suitable for use in an amorphous solid dispersion of the invention are, but are not limited to, homopolymers or copolymers of N-vinyl lactams, such as homopolymers or copolymers of N-vinyl pyrrolidone (e.g., polyvinylpyrrolidone (also named PVP or povidone), or copolymers of N-vinyl pyrrolidone and vinyl acetate or vinyl propionate); cellulose esters or cellulose ethers, such as alkylcelluloses (e.g., methylcellulose or ethylcellulose), hydroxyalkylcelluloses (e.g., hydroxypropylcellulose or hydroxyethylcellulose), hydroxyalkylalkylcelluloses (e.g., hydroxypropylmethylcellulose (also named HPMC)), and cellulose phthalates or succinates (e.g., cellulose acetate phthalate and hydroxypropylmethylcellulose phthalate (also named HPMCP), hydroxypropylmethylcellulose succinate, or hydroxypropylmethylcellulose acetate succinate); high molecular polyalkylene oxides, such as polyethylene oxide, polypropylene oxide, and copolymers of ethylene oxide and propylene oxide; polyacrylates or polymethacrylates, such as methacrylic acid/ethyl acrylate copolymers, methacrylic acid/methyl methacrylate copolymers, butyl methacrylate/2-dimethylaminoethyl methacrylate copolymers, poly(hydroxyalkyl acrylates), and poly(hydroxyalkyl methacrylates); polyacrylamides; vinyl acetate polymers, such as copolymers of vinyl acetate and crotonic acid, and partially hydrolyzed polyvinyl acetate (also referred to as partially saponified "polyvinyl alcohol"); polyvinyl alcohol; oligo- or polysaccharides, such as carrageenans, galactomannans, pectin, and xanthan gum; polyhydroxyalkylacrylates; polyhydroxyalkyl-methacrylates; copolymers of methyl methacrylate and acrylic acid; polyethylene glycols (PEGs); graft copolymers of polyethylene glycol/polyvinyl caprolactam/polyvinyl acetate, or any mixture or combination thereof.

Non-limiting examples of cellulose - based polymers are hydroxypropyl methylcellulose (HPMC) E3, HPMC E5, HPMC E6, HPMC E15, HPMC K3, HPMC A4, HPMC A15, HPMC acetate succinate (AS) LF, HPMC AS MF, HPMC AS HF, HPMC AS LG, HPMC AS MG, HPMC AS HG, HPMC phthalate (P) 50, HPMC P 55, Ethocel 4, Ethocel 7, Ethocel 10, Ethocel 14, Ethocel 20.

Non-limiting examples of polyethylene glycols are polyethylene glycol (PEG) 400, PEG 600, PEG 1450, PEG 3350, PEG 4000, PEG 6000, PEG 8000, poloxamer 124, poloxamer 188, poloxamer 237, poloxamer 338, and poloxamer 407.

Non-limiting examples of polyacrylates or polymethacrylates are methacrylate/methacrylic acid copolymer (Eudragit) L100-55, Eudragit L100, Eudragit S100.

According to a particular embodiment, the pharmaceutically acceptable carrier is a polymer.

According to a particular embodiment, the polymers suitable for use in an amorphous solid dispersion of the invention are selected from homopolymers of N-vinyl lactams, copolymers of N-vinyl lactams, and mixtures thereof.

In the sense of the present invention, the term « lactam » can include betalactam, gamma-lactam, delta lactam and epsilon-lactam, that is to say respectively a 4-membered, 5-membered, 6-membered and 7-membered carbon ring which includes one amide function.

As indicated above, among the homopolymers of N-vinyl lactams can be cited polyvinylpyrrolidone (also named povidone or PVP) which can be the ones sold for example by BASF under the name of Kollidon® 30 (also named PVP K30), PVP K17, PVP K25, PVP K30, or PVP K90.

As indicated above, among the copolymers of N-vinyl lactams can be cited copolymers of N-vinyl pyrrolidone and vinyl acetate (also named copovidone) which such as the one sold for example by BASF under the name of Kollidon® VA64 by BASF or copolymers of N-vinyl caprolactam, vinyl acetate, and ethylene glycol such as the one sold for example by BASF under the name of Soluplus®.

According to another particular embodiment, the polymers suitable for use in an amorphous solid dispersion of the invention are selected from povidone, copovidone, polyvinyl caprolactam - polyvinyl acetate - polyethylene glycol, and mixtures thereof.

According to another particular embodiment, the polymers suitable for use in an amorphous solid dispersion of the invention are selected from homopolymers of N-vinyl pyrrolidone, copolymers of N-vinyl pyrrolidone, and mixtures thereof.

Thus, according to one embodiment, in the amorphous solid dispersion according to the invention, the pharmaceutically acceptable carrier is a polymer which is selected from homopolymers of N-vinyl lactams, copolymers of N-vinyl lactams, and mixtures thereof, particularly from povidone, copovidone, polyvinyl caprolactam - polyvinyl acetate - polyethylene glycol, and mixtures thereof, more particularly from povidone, copovidone, and mixtures thereof, and still more particularly is copovidone.

According to a particular embodiment, the amorphous solid according to the invention is a glass solution forming a homogeneous one-phase system, and 8-chloro-N-(4-(trifluoromethoxy)phenyl)quinolin-2-amine or a pharmaceutically acceptable salt thereof is under an amorphous form.

According to a particular embodiment, the weight ratio of 8-chloro-N-(4-(trifluoromethoxy)phenyl)quinolin-2-amine or a pharmaceutically acceptable salt thereof and the pharmaceutically acceptable carrier(s) is in the range of from 1:20 to 1:0.5, particularly of from 1:10 to 1:1, more particularly of from 1:2 to 1:1.5.

It has to be understood that for the calculation of the weight ratio of 8-chloro-N-(4-(trifluoromethoxy)phenyl)quinolin-2-amine or a pharmaceutically acceptable salt thereof and the pharmaceutically acceptable carrier(s), it is taken into account only the amounts of ABX464 and of the pharmaceutically acceptable carrier(s) as defined in the invention which form an ASD according to the invention. In other terms, the calculation of this weight ratio does not consider the amounts of excipients which can be added afterwards so as to obtain a pharmaceutical composition according to the invention.

According to a particular embodiment, 8-chloro-N-(4-(trifluoromethoxy)phenyl)quinolin-2-amine or a pharmaceutically acceptable salt thereof is in an amount of from 5 % to 70% by weight, particularly of from 30 % to 40% by weight, more particularly from 33 % to 37% by weight, relative to the combined weight of 8-chloro-N-(4-(trifluoromethoxy)phenyl)quinolin-2-amine or a pharmaceutically acceptable salt thereof and the pharmaceutically acceptable carrier(s).

According to a particular embodiment, said pharmaceutically acceptable carrier(s) is(are) in an amount of from 30 % to 95% by weight, particularly of from 60 % to 70% by weight, more particularly from 63 % to 67% by weight relative to the combined weight of 8-chloro-N-(4-(trifluoromethoxy)phenyl)quinolin-2-amine or a pharmaceutically acceptable salt thereof and the pharmaceutically acceptable carrier(s).

According to a particular embodiment, the ASD in accordance with the invention comprises from 5% to 70% by weight of ABX464 or a pharmaceutically acceptable salt thereof and from 30% to 95% by weight of pharmaceutically acceptable carrier(s) relative to the combined weight of 8-chloro-N-(4-(trifluoromethoxy)phenyl)quinolin-2-amine or a pharmaceutically acceptable salt thereof and the pharmaceutically acceptable carrier(s).

According to a particular embodiment, the ASD in accordance with the invention comprises from 30% to 40% by weight of ABX464 or a pharmaceutically acceptable salt thereof and from 60% to 70% by weight of pharmaceutically acceptable carrier(s) relative to the combined weight of 8-chloro-N-(4-(trifluoromethoxy)phenyl)quinolin-2-amine or a pharmaceutically acceptable salt thereof and the pharmaceutically acceptable carrier(s).

According to a particular embodiment, the ASD in accordance with the invention comprises from 33% to 37% by weight of ABX464 or a pharmaceutically acceptable salt thereof and from 63% to 67% by weight of pharmaceutically acceptable carrier(s) relative to the combined weight of 8-chloro-N-(4-(trifluoromethoxy)phenyl)quinolin-2-amine or a pharmaceutically acceptable salt thereof and the pharmaceutically acceptable carrier(s).

According to a particular embodiment, the ASD in accordance with the invention comprises from 5% to 70% by weight of ABX464 or a pharmaceutically acceptable salt thereof and from 30% to 95% by weight of pharmaceutically acceptable polymer(s) relative to the combined weight of 8-chloro-N-(4-(trifluoromethoxy)phenyl)quinolin-2-amine or a pharmaceutically acceptable salt thereof and the pharmaceutically acceptable polymer(s).

According to a particular embodiment, the ASD in accordance with the invention comprises from 30% to 40% by weight of ABX464 or a pharmaceutically acceptable salt thereof and from 60% to 70% by weight of pharmaceutically acceptable polymer(s) relative to the combined weight of 8-chloro-N-(4-(trifluoromethoxy)phenyl)quinolin-2-amine or a pharmaceutically acceptable salt thereof and the pharmaceutically acceptable polymer(s).

According to a particular embodiment, the ASD in accordance with the invention comprises from 33% to 37% by weight of ABX464 or a pharmaceutically acceptable salt thereof and from 63% to 67% by weight of pharmaceutically acceptable polymer(s) relative to the combined weight of 8-chloro-N-(4-(trifluoromethoxy)phenyl)quinolin-2-amine or a pharmaceutically acceptable salt thereof and the pharmaceutically acceptable polymer(s).

According to a particular embodiment, the ASD in accordance with the invention comprises from 5% to 70% by weight of ABX464 or a pharmaceutically acceptable salt thereof and from 30% to 95% by weight of pharmaceutically acceptable polymer(s) which is selected from homopolymers of N-vinyl lactams, copolymers of N-vinyl lactams, and mixtures thereof, relative to the combined weight of 8-chloro-N-(4-(trifluoromethoxy)phenyl)quinolin-2-amine or a pharmaceutically acceptable salt thereof and the pharmaceutically acceptable polymer(s).

According to a particular embodiment, the ASD in accordance with the invention comprises from 30% to 40% by weight of ABX464 or a pharmaceutically acceptable salt thereof and from 60% to 70% by weight of pharmaceutically acceptable polymer(s) which is selected from homopolymers of N-vinyl lactams, copolymers of N-vinyl lactams, and mixtures thereof, relative to the combined weight of 8-chloro-N-(4-(trifluoromethoxy)phenyl)quinolin-2-amine or a pharmaceutically acceptable salt thereof and the pharmaceutically acceptable polymer(s).

According to a particular embodiment, the ASD in accordance with the invention comprises from 33% to 37% by weight of ABX464 or a pharmaceutically acceptable salt thereof and from 63% to 67% by weight of pharmaceutically acceptable polymer(s) which is selected from homopolymers of N-vinyl lactams, copolymers of N-vinyl lactams, and mixtures thereof, relative to the combined weight of 8-chloro-N-(4-(trifluoromethoxy)phenyl)quinolin-2-amine or a pharmaceutically acceptable salt thereof and the pharmaceutically acceptable polymer(s).

According to a particular embodiment, the ASD in accordance with the invention comprises from 5% to 70% by weight of ABX464 or a pharmaceutically acceptable salt thereof and from 30% to 95% by weight of pharmaceutically acceptable polymer(s) which is selected from povidone, copovidone, polyvinyl caprolactam - polyvinyl acetate - polyethylene glycol, and mixtures thereof, relative to the combined weight of 8-chloro-N-(4-(trifluoromethoxy)phenyl)quinolin-2-amine or a pharmaceutically acceptable salt thereof and the pharmaceutically acceptable polymer(s).

According to a particular embodiment, the ASD in accordance with the invention comprises from 30% to 40% by weight of ABX464 or a pharmaceutically acceptable salt thereof and from 60% to 70% by weight of pharmaceutically acceptable polymer(s) which is selected from povidone, copovidone, polyvinyl caprolactam - polyvinyl acetate - polyethylene glycol, and mixtures thereof, relative to the combined weight of 8-chloro-N-(4-(trifluoromethoxy)phenyl)quinolin-2-amine or a pharmaceutically acceptable salt thereof and the pharmaceutically acceptable polymer(s).

According to a particular embodiment, the ASD in accordance with the invention comprises from 33% to 37% by weight of ABX464 or a pharmaceutically acceptable salt thereof and from 63% to 67% by weight of pharmaceutically acceptable polymer(s) which is selected from povidone, copovidone, polyvinyl caprolactam - polyvinyl acetate - polyethylene glycol, and mixtures thereof, relative to the combined weight of 8-chloro-N-(4-(trifluoromethoxy)phenyl)quinolin-2-amine or a pharmaceutically acceptable salt thereof and the pharmaceutically acceptable polymer(s).

According to a particular embodiment, the ASD in accordance with the invention comprises from 30% to 40% by weight of ABX464 or a pharmaceutically acceptable salt thereof and from 60% to 70% by weight of povidone relative to the combined weight of 8-chloro-N-(4-(trifluoromethoxy)phenyl)quinolin-2-amine or a pharmaceutically acceptable salt thereof and povidone.

According to a particular embodiment, the ASD in accordance with the invention comprises from 33% to 37% by weight of ABX464 or a pharmaceutically acceptable salt thereof and from 63% to 67% by weight of povidone relative to the combined weight of 8-chloro-N-(4-(trifluoromethoxy)phenyl)quinolin-2-amine or a pharmaceutically acceptable salt thereof and povidone.

According to a particular embodiment, the ASD in accordance with the invention comprises from 30% to 40% by weight of ABX464 or a pharmaceutically acceptable salt thereof and from 60% to 70% by weight of copovidone relative to the combined weight of 8-chloro-N-(4-(trifluoromethoxy)phenyl)quinolin-2-amine or a pharmaceutically acceptable salt thereof and copovidone.

According to a particular embodiment, the ASD in accordance with the invention comprises from 33% to 37% by weight of ABX464 or a pharmaceutically acceptable salt thereof and from 63% to 67% by weight of copovidone relative to the combined weight of 8-chloro-N-(4-(trifluoromethoxy)phenyl)quinolin-2-amine or a pharmaceutically acceptable salt thereof and copovidone.

According to a particular embodiment, the ASD in accordance with the invention comprises from 30% to 40% by weight of ABX464 or a pharmaceutically acceptable salt thereof and from 60% to 70% by weight of polyvinyl caprolactam - polyvinyl acetate - polyethylene glycol relative to the combined weight of 8-chloro-N-(4-(trifluoromethoxy)phenyl)quinolin-2-amine or a pharmaceutically acceptable salt thereof and polyvinyl caprolactam - polyvinyl acetate - polyethylene glycol.

According to a particular embodiment, the ASD in accordance with the invention comprises from 33% to 37% by weight of ABX464 or a pharmaceutically acceptable salt thereof and from 63% to 67% by weight of polyvinyl caprolactam - polyvinyl acetate - polyethylene glycol relative to the combined weight of 8-chloro-N-(4-(trifluoromethoxy)phenyl)quinolin-2-amine or a pharmaceutically acceptable salt thereof and polyvinyl caprolactam - polyvinyl acetate - polyethylene glycol.

It has to be understood that for the calculation of all these percentages by weight relative to the combined weight of 8-chloro-N-(4-(trifluoromethoxy)phenyl)quinolin-2-amine or a pharmaceutically acceptable salt thereof and of the pharmaceutically acceptable carrier(s) as defined in the invention, it is taken into account only the amounts of ABX464 and of the pharmaceutically acceptable carrier(s) as defined in the invention which form an ASD according to the invention. In other terms, the calculation of these percentages by weight does not consider the amounts of excipients which can be added afterwards so as to obtain a pharmaceutical composition according to the invention.

The pharmaceutically acceptable carrier suitable for the present invention can be a combination of two, of three, of four, of five or more of a compound chosen among a polymer, a sugar, an acid, a surfactant, a cyclodextrin, pentaerythritol, pentaerythrityl tetraacetate, urea, urethane, and hydroxy alkyl xanthins.

According to a particular embodiment, said pharmaceutically acceptable carrier is a combination of polymer(s) as defined in the invention and of acid(s) as defined in the invention, particularly citric acid, succinic acid, malic acid, fumaric acid tartaric acid or mixtures thereof.

According to another particular embodiment, said pharmaceutically acceptable carrier is a combination of polymer(s) as defined in the invention and of sugar(s) as defined in the invention.

According to another particular embodiment, said pharmaceutically acceptable carrier is a combination of polymer(s) as defined in the invention and of surfactant(s) as defined in the invention.

According to another particular embodiment, said pharmaceutically acceptable carrier is a combination of polymer(s) as defined in the invention and of cyclodextrin(s) as defined in the invention.

According to another particular embodiment, said pharmaceutically acceptable carrier is a combination of polymer(s) as defined in the invention and of pentaerythritol.

According to another particular embodiment, said pharmaceutically acceptable carrier is a combination of polymer(s) as defined in the invention and of pentaerythrityl tetraacetate.

According to another particular embodiment, said pharmaceutically acceptable carrier is a combination of polymer(s) as defined in the invention and of urea.

According to another particular embodiment, said pharmaceutically acceptable carrier is a combination of polymer(s) as defined in the invention and of urethane.

According to another particular embodiment, said pharmaceutically acceptable carrier is a combination of polymer(s) as defined in the invention and of hydroxy alkyl xanthins.

As indicated above, depending on the nature of the pharmaceutical carrier, three different types (or categories) of ASD can be observed. Indeed, a pharmaceutical carrier suitable for use in an ASD in accordance with the present invention may possess either one or both of the following aspects: strong intermolecular interactions and low molecular mobility.

Strong intermolecular interactions between the molecules of the carrier(s) and the molecule of ABX464 allow to stabilize and to maintain ABX464 under an amorphous form and to prevent the molecules from coming together.

The ASD according to the invention are thermally stable, stay under amorphous form, even under stress conditions as detailed below, form homogeneous one - phase system (see for instance the below examples 2.2, 3 and 4). As mentioned above, it has also been proved in the experimental part by using Fasted and Fed Human in vitro models that the amorphous form of ABX464 in the ASD can be kept after the administration of the product in a subject in need (patient) thereof and that the ASD in accordance with the present invention present a significantly more important solubility compared to ABX464 in its unique crystalline form (see example 3). In addition, the inventors have demonstrated that the ASD according to the invention are chemically and physically stable after 2 weeks under stress conditions (at a temperature of 25°C and 60% relative humidity; and also at a temperature of 40°C and 75% relative humidity) as shown in example 4.

## PROCESS FOR THE PREPARATION OF ASD ACCORDING TO THE INVENTION

As mentioned above, herein is also provided a process for the preparation of the amorphous solid dispersion as defined in the present invention, comprising the following step:
aa) combining 8-chloro-N-(4-(trifluoromethoxy)phenyl)quinolin-2-amine or a pharmaceutically acceptable salt thereof and at least one pharmaceutically acceptable carrier as defined in the present invention, optionally in a suitable solvent or mixture of solvents so as to obtain a solution to provide the amorphous solid dispersion.

Herein is also provided, according to another embodiment, a process for the preparation of the amorphous solid dispersion as defined in the present invention, comprising the following steps:

aa) combining 8-chloro-N-(4-(trifluoromethoxy)phenyl)quinolin-2-amine or a pharmaceutically acceptable salt thereof and at least one pharmaceutically acceptable carrier as defined in the present invention, optionally in a suitable solvent or mixture of solvents so as to obtain a solution;
bb) mixing the combination or solution obtained in step aa); and
cc) optionally evaporating the solvent(s) to provide the amorphous solid dispersion.

Herein is also provided, according to another embodiment, a process for the preparation of the amorphous solid dispersion as defined in the present invention, comprising the following steps:

a) Dissolving 8-chloro-N-(4-(trifluoromethoxy)phenyl)quinolin-2-amine or a pharmaceutically acceptable salt thereof in a suitable solvent or mixture of solvents so as to obtain a solution;
b) Adding to the thus obtained solution of step a) at least one pharmaceutically acceptable carrier as defined in the present invention;
c) Optionally mixing the mixture obtained in step b); and
d) Evaporating the solvent(s) to provide the amorphous solid dispersion.

In step a), the suitable solvent can be any volatile solvent which is able to dissolve both the pharmaceutically acceptable carrier and ABX464 or a salt thereof, particularly the suitable solvent is selected from a $C_1$-$C_6$ alcohol, dichloromethane, acetonitrile, acetone, THF (tetrahydrofuran), diethyl ether and mixtures thereof, more particularly is selected from a $C_1$-$C_4$ monoalcohol, dichloromethane and mixtures thereof, still more particularly is selected from methanol, ethanol, dichloromethane, and mixtures thereof, even more particularly is methanol.

Advantageously, because of the higher viscosity of the glass solutions in accordance with the present invention as compared to liquid solutions, the distribution of solute molecules (ABX 464 molecules) may be irregular in the pharmaceutically acceptable carrier and a homogeneous distribution within the glass solution can be ensured by mixing (step c). This mixing can be carried out by any conventional means known in the art.

The evaporating step d) can be carried out by spray - drying, holt-melt extrusion, or solvent evaporation method, particularly by spray - drying or holt-melt extrusion, more particularly by spray - drying. These methods are well-known in the art (see for example Prashant et al., Amorphous solid dispersion: a promising technique for improving oral bioavailability of poorly water-soluble drugs, S. Afr. Pharm. J., 2018, 85(1), 50-56).

When spray-drying evaporation step is used, the ASD in accordance with the invention can also be named in the present text spray dried dispersion (SDD).

Spray - drying comprises 3 well-known steps: atomization, drying and collection of the powder. Typically, the spraying is carried out via a nozzle to obtain the ASD. Some of the parameters are generally as follows:

- the nozzle can be from 0.4 mm to 1 mm, particularly is 0.6 mm;
- the inlet temperature can be from 85 °C to 100 °C, particularly is 90°C;
- the outlet temperature can be from 56 °C to 57 °C (this temperature being a consequence of a combination of parameters);
- the flow rate can be from 2 mL/min to 5 mL/min, particularly is 3 mL/min;
- the nozzle flow rate can be from 6 L/h to 10 L/h, particularly is 8 L/h.

In holt-melt extrusion, ABX464 or a salt thereof is melted or dissolved within a dispersion pharmaceutically acceptable carrier as defined in the invention and is mixed to produce and stabilize the amorphous form of ABX464 or a salt thereof. The melt is then extruded and rapidly cooled to obtain a stable solid single - phase glassy amorphous matrix. If needed, the thus obtained product can then be advantageously milled to reduce the particle size and then can be introduced into an oral solid dosage form such as a tablet or a capsule as defined herein.

In solvent evaporation method, the ABX 464 or a salt thereof and said pharmaceutically acceptable carrier as defined in the invention are solubilized in a volatile solvent. Advantageously, a mixing at the molecular level can be carried out (to optimize the dissolution properties of the final product). When this method is used, care should be taken during the mixing of both ABX464 and the carrier in one solution and to avoid phase separation.

According to a particular embodiment, a process for the preparation of the amorphous solid dispersion as defined in the present invention, comprises the following steps:

> a) Dissolving 8-chloro-N-(4-(trifluoromethoxy)phenyl)quinolin-2-amine or a pharmaceutically acceptable salt thereof in methanol, ethanol or dichloromethane so as to obtain a solution;
> b) Adding to the thus obtained solution of step a) povidone, copovidone or polyvinyl caprolactam - polyvinyl acetate - polyethylene glycol;
> c) Optionally mixing the mixture obtained in step b); and
> d) Evaporating methanol to provide the amorphous solid dispersion.

According to another particular embodiment, a process for the preparation of the amorphous solid dispersion as defined in the present invention, comprises the following steps:

> a) Dissolving 8-chloro-N-(4-(trifluoromethoxy)phenyl)quinolin-2-amine or a pharmaceutically acceptable salt thereof in methanol so as to obtain a solution;
> b) Adding to the thus obtained solution of step a) povidone, copovidone or polyvinyl caprolactam - polyvinyl acetate - polyethylene glycol;
> c) Optionally mixing the mixture obtained in step b); and
> d) Evaporating methanol to provide the amorphous solid dispersion.

The process of preparation in accordance with the invention presents many advantages.

First, the process comprises few (three) essential steps.

It also allows to obtain the ASD in significantly high yields (at least about 80%).

In addition, it allows to prepare ASD in accordance with the present invention which are thermally stable up to 100°C (that is to say ASD stay into amorphous form) and homogeneous, as shown in the experimental part (XRPD, and mDSC characterizations, see example 2.2).

Furthermore, the UV-HPLC assay as explained in the experimental part (see example 2.1) demonstrates that the process allows to maintain the load of ABX464 in the ASD after the evaporation step d), more particularly after the spray-drying step.

## PHARMACEUTICAL COMPOSITIONS ACCORDING TO THE INVENTION

Herein is also provided a pharmaceutical composition comprising the amorphous solid dispersion as defined in the invention, and at least one pharmaceutically acceptable excipient.

According to a particular embodiment, a pharmaceutical composition in accordance with the invention is an oral pharmaceutical composition.

Oral pharmaceutical composition of the present invention can be in the form of capsules, tablets, or sachets comprising the composition in powder form. A therapeutically effective oral dosage for formulations of the invention is determined by standard clinical techniques according to the judgment of a medical practitioner.

Due to the fact that the ASD according to the invention are obtained under the form of powder, it is advantageous to protect them from relative humidity.

Thus, according to one embodiment, when the ASD of the present invention are formulated into capsules or tablets by using conventional methods, the are protected in blisters. Another advantage conferred by the use of blisters is that the capsules or tablets are also protected from oxygen and other contaminants.

In some embodiments, the present invention provides a tablet or a capsule comprising the amorphous solid dispersion of the present invention, and at least one pharmaceutically acceptable excipient.

The excipients can be any conventional used excipients including intragranular excipients, and/or an extragranular excipients.

The excipients may be selected from fillers, binders, antioxidants, disintegrants, lubricants, glidants, surfactants (distinct from the surfactants used as pharmaceutically acceptable carriers in the ASD), and mixtures thereof.

Fillers which are useable in accordance with the invention include, but are not limited to, lactose (anhydrous), lactose monohydrate, spray-dried lactose; compressible sugar, dextrose, dextrates; starches (including starches from any source, such as corn, potato, rice, wheat, which can be fully pregelatinized and partially gelatinized); cellulose; microcrystalline cellulose ; inorganic salts such as calcium phosphate, tribasic calcium and calcium sulfate; and polyols such as mannitol, sorbitol and xylitol.

In some embodiments, the filler may be in an amount of 10% to 85% by weight based on the total weight of the composition.

Lubricants which are useable according to the invention include, but are not limited to, magnesium stearate, calcium stearate, zinc stearate, stearic acid, sodium stearyl fumarate, hydrogenated vegetable oils, mineral oil, polyethylene glycols, talc, glyceryl behenate, glyceryl monostearate, glyceryl palmitostearate, leucine, and magnesium lauryl sulfate.

In some embodiments, the lubricant may be in an amount of 0.3% to 2% by weight based on the total weight of the composition.

Disintegrants which are useable in accordance with the invention include, but are not limited to, croscarmellose sodium, sodium starch glycolate, starches (including starches from any source, such as corn, potato, rice, wheat, fully pregelatinized and partially gelatinized), crospovidone, alginates such as calcium alginate and sodium alginate, alginic acid, and magnesium aluminum silicate.

In some embodiments, the disintegrant may be in an amount of 30% to 60% by weight based on the total weight of composition.

The surfactants employable as an additive in the present invention include, but are not limited to, tocopherol, lecithin, egg yolk phosphatides, docusate sodium, Capryol, Labrafil, Labrasol, Lauroglycol, and mixtures thereof.

In some embodiments, the surfactant may be in an amount of 1% to 3% by weight based on the total weight of composition.

In some embodiments, the glidant may be in an amount of 0.3% to 2% by weight based on the total weight of composition.

In some embodiments, the binder may be in an amount of 5% to 20% by weight based on the total weight of composition.

According to a particular embodiment, the pharmaceutical composition according to the invention comprises 8-chloro-N-(4-(trifluoromethoxy)phenyl)quinolin-2-amine or a pharmaceutically acceptable salt thereof as the sole pharmaceutically active ingredient.

According to a particular embodiment, 8-chloro-N-(4-(trifluoromethoxy)phenyl)quinolin-2-amine or a pharmaceutically acceptable salt thereof is in an amount of from 5 % to 95% by weight, relative to the total weight of the pharmaceutical composition.

According to a particular embodiment, said pharmaceutically acceptable carrier(s) is(are) in an amount of from 5 % to 95% by weight, relative to the total weight of the pharmaceutical composition.

In some embodiments, the pharmaceutically acceptable excipients can include one or more of the above-defined pharmaceutically acceptable carrier(s).

According to a particular embodiment, a pharmaceutical composition according to the invention comprises a ABX464: VA64 ASD or a ABX464: K30 ASD as defined in the invention and further excipient(s) such as pharmaceutically acceptable polymer(s), in particular copovidone and/or povidone. It has to be understood that in these cases, the amount of polymers (for example copovidone and/or povidone) considered as excipients has not to be considered in the calculation of the weight ratio ABX464/pharmaceutically acceptable carrier(s) as defined in the present invention for the ASD in accordance with the invention and has not to be taken into account in the determination of the amount by weight relative to the combined weight of ABX464 and the pharmaceutically acceptable carrier(s) as defined for the ASD in accordance with the invention.

According to one embodiment, a pharmaceutical composition in accordance with the invention is such that a dose of from 1 mg to 1 g per day, particularly of from 10 mg to 150 mg per day of active ingredient ABX464 is administered to a subject in need thereof in one or more doses per day.

## PROCESS FOR THE PREPARATION OF PHARMACEUTICAL COMPOSITION ACCORDING TO THE INVENTION

As mentioned above, herein is also provided a process for the preparation of the pharmaceutical composition as defined in the invention, comprising the following steps:

a) Dissolving 8-chloro-N-(4-(trifluoromethoxy)phenyl)quinolin-2-amine or a pharmaceutically acceptable salt thereof in a suitable solvent or mixture of solvents so as to obtain a solution;

b) Adding to the thus obtained solution of step a) at least one pharmaceutically acceptable carrier as defined in the present invention;

c) Optionally mixing the mixture obtained in step b);

d) Evaporating the solvent(s) to provide the amorphous solid dispersion;

e) Mixing together the amorphous solid dispersion of step d) with excipient(s) to obtain the pharmaceutical composition; and

f) Optionally coating the thus obtained pharmaceutical composition when a coated pharmaceutical composition is needed.

Steps a), b), c) and d) are the same as the ones defined above for the process of preparation of ASD according to the invention.

The step e) of mixing can be carried out by any conventional means known in the art.

The step f) of coating can be performed by using any conventional coating agent(s) known in the art.

When the pharmaceutical composition of the invention is in the form of a tablet, a step of compressing the mixture as obtained in step e) has to be carried out between step e) and optional step f).

## THERAPEUTIC USES AND METHOD OF ADMINISTRATION

As mentioned above, herein are also provided an amorphous solid dispersion as defined in the invention or a pharmaceutical composition as defined in the present invention for use as a medicament and for use in the treatment and / or prevention of inflammatory diseases such as Inflammatory Bowel Disease, Rheumatoid Arthritis, pulmonary arterial hypertension, NASH and Multiple Sclerosis, diseases caused by viruses and/or cancer or dysplasia.

A method for administering ABX464 or a salt thereof to a subject in need thereof is provided, comprising:

- providing an oral pharmaceutical composition comprising: ABX464 or a pharmaceutically acceptable salt thereof, and at least one pharmaceutically acceptable carrier prepared as an ASD, and at least one pharmaceutically acceptable excipient; and

- orally administering the pharmaceutical composition in a therapeutically effective amount to a subject in need thereof.

ABX464 or a salt thereof can be administered alone or in combination with other therapeutic agents which can act synergistically with ABX464 or a salt thereof.

In further embodiments, the invention encompasses methods of orally administering a pharmaceutical composition comprising the amorphous solid dispersion containing ABX464 or a salt thereof, and an additional therapeutic agent.

A "subject" (including patient) includes mammals, e.g., humans, companion animals (e.g., dogs, cats, birds, and the like), farm animals (e.g., cows, sheep, pigs, horses, fowl, and the like) and laboratory animals (e.g., rats, mice, guinea pigs, birds, and the like).

According to another aspect, the present invention also relates to the use of an amorphous solid dispersion as defined in the invention or a pharmaceutical composition as defined in the present invention for the manufacture of a medicament.

According to another aspect, the present invention also relates to the use of an amorphous solid dispersion as defined in the invention or a pharmaceutical composition as defined in the present invention for the manufacture of a medicament for preventing and/or treating inflammatory diseases such as Inflammatory Bowel Disease, Rheumatoid Arthritis, pulmonary arterial hypertension, NASH and Multiple Sclerosis, diseases caused by viruses and/or cancer or dysplasia.

According to another aspect, the present invention also relates to a therapeutic method of treating and/ or preventing inflammatory diseases such as Inflammatory Bowel Disease, Rheumatoid Arthritis, pulmonary arterial hypertension, NASH and Multiple Sclerosis, diseases caused by viruses and/or cancer or dysplasia comprising administering to a patient in need thereof a pharmaceutical composition comprising an ASD as defined in the present invention.

According to another aspect, the present invention also relates to a therapeutic method of treating and/or preventing inflammatory diseases such as Inflammatory Bowel Disease, Rheumatoid Arthritis, pulmonary arterial hypertension, NASH and Multiple Sclerosis, diseases caused by viruses and/or cancer or dysplasia comprising administering to a patient in need thereof a therapeutically effective amount of an ASD as defined in the present invention.

### Inflammatory diseases

Thus, the invention also relates to an ASD as defined above or a pharmaceutical composition as defined in the present invention, for use in the treatment and/or prevention of an inflammatory disease.

According to the invention, an « *inflammation* » is a protective response by the immune system to tissue damage and infection. However, the inflammatory response, in some circumstances, can damage the body. In the acute phase, inflammation is characterized by pain, heat, redness, swelling and loss of function. Inflammation can result from infection, irritation, or injury.

Thus, an « *inflammatory disease* » refers to a group of diseases and/or disorders that are caused by an excessive or dysregulated inflammation.

In a non-limitative manner, inflammatory diseases include: an inflammatory disease associated with an autoimmune disease, a central nervous system (CNS) inflammatory disease, a joint inflammation disease, an inflammatory digestive tract disease, inflammatory skin and other inflammatory diseases related to epithelial cells such as bronchitis, inflammation associated with cancer, such as colon carcinoma, inflammation associated with irritation, and inflammation associated with injury.

According to the present invention, the inflammatory disease, disorder or condition is selected from:

(a) an inflammatory disease, disorder, or condition in the pancreas selected from diabetes type-1, diabetes type-2, acute and chronic pancreatitis;
(b) an inflammatory disease, disorder, or condition in the kidney selected from glomerulosclerosis, glomerulonephritis, nephritis, acute kidney injury, Berger's disease, Goodpasture's syndrome, Wegener's granulomatosis and kidney transplant acute or chronic rejection;
(c) an inflammatory disease, disorder, or condition in the liver selected from nonalcoholic steatohepatitis (NASH), non-alcoholic fatty liver disease (NAFLD), cholestatic liver disease, sclerosing cholangitis and liver transplant acute or chronic rejection;
(d) an inflammatory disease, disorder, or condition in the lung or heart selected from chronic obstructive pulmonary disease (COPD), asthma, pulmonary fibrosis, pulmonary arterial hypertension, sarcoidosis, myocarditis, pericarditis and lung or heart transplant acute or chronic rejection;
(e) an inflammatory disease, disorder, or condition in the skin selected from contact dermatitits, atopic dermatitis, urticaria, chronic dermatitis, psoriasis, eczema, alopecia areata, erythema multiforma, dermatitis herpetiformis, scleroderma, vitiligo, hypersensitivity angiitis, urticaria, bullous pemphigoid, pemphigus vulgaris, pemphigus foliaceus, paraneoplastic pemphigus, epidermolysis bullosa acquisita, acnea, keloid scar, and other inflammatory or allergic conditions of the skin;
(f) an inflammatory disease, disorder, or condition in the vessel/blood selected from Behcet's disease, vasculitis, sepsis, tumor angiogenesis, atherosclerosis, proliferative vascular disease and restenosis;
(g) an inflammatory disease, disorder, or condition in the eye selected from conjunctivitis, scleritis, episcleritis, panuveitis, choroiditis, chorioretinitis, neuroretinitis, uveitis, orbital inflammatory disease, and optical neuritis;
(h) an inflammatory disease, disorder, or condition in the central or peripheral nervous system selected from non-viral and viral encephalitis and meningitis, depression, neuropathic pain, chronic pain, traumatic brain injury, including stroke, Alzheimer disease, Parkinson disease, Myelitis, Charcot-Marie-Tooth disease type 1 (including CMT1A and CMT1B), Multiple Sclerosis, Amyotrophic lateral sclerosis (ALS), Creutzfeldt-Jakob disease, demyelinating polyneuropathy and peripheral neuropathy;
(i) an autoimmune disease, disorder, or condition selected from Lupus, including in the skin and kidney, Guillain-Barre syndrome, Myasthenia gravis, Hashimoto's thyroiditis, idiopathic purpura, aplastic anemia, Graves disease, and Myocarditis;
(j) an inflammatory disease, disorder, or condition in the intestine selected from intestinal failure, Ulcerative colitis (UC) and Crohn's disease,
(k) an inflammatory disease, disorder, or condition in the reproductive system selected from endometriosis, uterine fibroma, prostate dysplasia or growth, and cervix dysplasia; and
(l) an inflammatory disease, disorder, or condition in the bone and/or joints selected from rheumatoid arthritis, juvenile idiopathic arthritis, psoriatic arthritis, periodontitis, and hand, foot, ankle, knee, hip, shoulder, elbow or spine arthritis and/or demineralization.

In a particular embodiment, the inflammatory disease can be selected in the list consisting of: an inflammatory disease associated with an autoimmune disease, a central nervous system (CNS) inflammatory disease, a joint inflammation disease, an inflammatory digestive tract disease, inflammatory skin and other inflammatory diseases related to epithelial cells, inflammation associated with cancer, inflammation associated with irritation, and inflammation associated with injury.

In particular, an inflammatory disease is selected in the list consisting of: Inflammatory Bowel Disease, Rheumatoid Arthritis, Crohn's disease, Ulcerative Colitis, Multiple Sclerosis, Alzheimer's disease, Parkinson, osteoarthritis, atherosclerosis, ankylosing spondylitis, psoriasis, dermatitis, Sjogren's syndrom, bronchitis, asthma, pulmonary arterial hypertension, NASH and inflammation associated with colon carcinoma.

More particularly, an inflammatory disease is selected in the list consisting of: Inflammatory Bowel Disease, Rheumatoid Arthritis, Crohn's disease, Ulcerative Colitis, Multiple Sclerosis, osteoarthritis, ankylosing spondylitis, psoriasis, Sjogren's syndrom, bronchitis, pulmonary arterial hypertension, NASH and inflammation associated with colon carcinoma.

More particularly, an inflammatory disease is selected in the list consisting of: Inflammatory Bowel Disease, Rheumatoid Arthritis, Crohn's disease, Ulcerative Colitis, Multiple Sclerosis, osteoarthritis, ankylosing spondylitis, pulmonary arterial hypertension, NASH and psoriasis.

Preferably, an inflammatory disease according to the invention includes: Inflammatory Bowel Disease, Crohn's disease, Ulcerative Colitis, Rheumatoid Arthritis, pulmonary arterial hypertension, NASH and Multiple Sclerosis.

Even more preferably, an inflammatory disease according to the invention includes: Inflammatory Bowel Disease, Rheumatoid Arthritis, pulmonary arterial hypertension, NASH and Multiple Sclerosis.

An inflammatory disease may also encompass Alzheimer's disease, Parkinson, asthma, atherosclerosis and dermatitis.

As dermatitis, eczema may be cited.

In view of the above, the invention relates to an ASD as defined in the present invention or a pharmaceutical composition as defined in the present invention for use in the treatment and/or prevention of an inflammatory disease, which encompasses inflammation as such, and inflammation associated with an inflammatory disease.

Thus, the invention also relates to the use of an ASD as defined in the present invention or a pharmaceutical composition as defined in the present invention for treating and/or preventing an inflammatory disease, which encompasses inflammation as such, and inflammation associated with an inflammatory disease.

The invention also relates to the use of an ASD as defined in the present invention for the preparation of a composition, such as a medicament, for treating and/or preventing inflammation, which encompasses inflammation as such, and inflammation associated with an inflammatory disease.

The invention also relates to a method for treating and/or preventing an inflammatory disease, which includes inflammation as such, and inflammation associated with said inflammatory disease, and which comprises a step of administering an ASD as defined in the present invention or a pharmaceutical composition as defined in the present invention to a patient in need thereof.

In some embodiments, a method of the present invention or an ASD as defined in the present invention or a pharmaceutical composition as defined in the present invention for use as defined above, for treating an inflammatory disease, disorder or condition further comprises measuring and/or monitoring a presence and/or level of a biomarker in a patient, for example in a blood, plasma, tissue, saliva, and/or serum sample. In some embodiments, a biomarker measured and/or monitored in a method of the present invention is miR-124.

In some embodiments, a method of the present invention or an ASD as defined in the present invention or a pharmaceutical composition as defined in the present invention for use as defined above, for treating an inflammatory disease, disorder or condition further comprises measuring and/or monitoring a presence and/or expression level of miR-124 in a patient, for example in a blood, plasma, tissue, saliva, and/or serum sample, prior to administering an ASD as defined in the present invention or a pharmaceutical composition as defined in the present invention, as described herein.

In some embodiments, a method of the present invention or an ASD as defined in the present invention or a pharmaceutical composition as defined in the present invention for use as defined above, for treating an inflammatory disease, disorder or condition further comprises measuring and/or monitoring a presence and/or expression level of miR-124 in a patient during the course of a treatment with an ASD as defined in the present invention or a pharmaceutical composition as defined in the present invention thereof as described herein.

In some embodiments, a method of the present invention for treating an inflammatory disease, disorder or condition, or an ASD as defined in the present invention or a pharmaceutical composition as defined in the present invention for use as defined above, further comprises selecting a patient for a treatment with an ASD as defined in the present invention or a pharmaceutical composition as defined in the present invention thereof as described herein, by measuring and/or monitoring a presence and/or expression level of miR-124 in the patient.

A provided ASD can be administered alone or in combination with one or more other therapeutic compounds, possible combination therapy taking the form of fixed combinations or the administration of a compound of the invention and one or more other therapeutic compounds being staggered or given independently of one another, or the combined administration of fixed combinations and one or more other therapeutic compounds. A compound of the current invention can besides or in addition be administered especially for tumor therapy in combination with chemotherapy, radiotherapy, immunotherapy, phototherapy, surgical intervention, or a combination of these. Long-term therapy is equally possible as is adjuvant therapy in the context of other treatment strategies, as described above. Other possible treatments are therapy to maintain the patient's status after tumor regression, or even chemopreventive therapy, for example in patients at risk.

Those additional agents may be administered separately from a provided ASD, as part of a multiple dosage regimen. Alternatively, those agents may be part of a single dosage form, mixed together with a provided ASD in a single composition. If administered as part of a multiple dosage regime, the two active agents may be submitted simultaneously, sequentially or within a period of time from one another normally within five hours from one another.

As used herein, the term "combination," "combined," and related terms refers to the simultaneous or sequential administration of therapeutic agents in accordance with this invention. For example, a provided ASD may be administered with another therapeutic agent simultaneously or sequentially in separate unit dosage forms or together in a single unit dosage form. Accordingly, the present invention provides a single unit dosage form comprising a provided ASD, an additional therapeutic agent, and a pharmaceutically acceptable carrier, adjuvant, or vehicle.

In some embodiments, the ASD as defined in the present invention may be administered with one or more additional therapeutic agents. Such additional therapeutic agents may be small molecules or recombinant biologic agents and include, for example, acetaminophen, non-steroidal anti-inflammatory drugs (NSAIDS) such as aspirin, ibuprofen, naproxen, etodolac (Lodine®) and celecoxib, colchicine (Colcrys®), corticosteroids such as prednisone, prednisolone, methylprednisolone, hydrocortisone, and the like, probenecid, allopurinol, febuxostat (Uloric®), sulfasalazine (Azulfidine®), antimalarials such as hydroxychloroquine (Plaquenil®) and chloroquine (Aralen®), methotrexate (Rheumatrex®), gold salts such as gold thioglucose (Solganal®), gold thiomalate (Myochrysine®) and auranofin (Ridaura®), D-penicillamine (Depen® or Cuprimine®), azathioprine (Imuran®), cyclophosphamide (Cytoxan®), chlorambucil (Leukeran®), cyclosporine (Sandimmune®, Neoral®), tacrolimus, sirolimus, mycophenolate, leflunomide (Arava®) and "anti-TNF" agents such as etanercept (Enbrel®), infliximab (Remicade®), golimumab (Simponi®), certolizumab pegol (Cimzia®) and adalimumab (Humira®), "anti-IL-1" agents such as anakinra (Kineret®) and rilonacept (Arcalyst®), anti-T cell antibodies such as Thymoglobulin, IV Immunoglobulins (IVIg), canakinumab (Ilaris®), anti-Jak inhibitors such as tofacitinib, antibodies such as rituximab (Rituxan®), "anti-T-cell" agents such as abatacept (Orencia®), "anti-IL-6" agents such as tocilizumab (Actemra®), diclofenac, cortisone, hyaluronic acid (Synvisc® or Hyalgan®), monoclonal antibodies such as tanezumab, anticoagulants such as heparin (Calcinparine® or Liquaemin®) and warfarin (Coumadin®), antidiarrheals such as diphenoxylate (Lomotil®) and loperamide (Imodium®), bile acid binding agents such as cholestyramine, alosetron (Lotronex®), lubiprostone (Amitiza®), laxatives such as Milk of Magnesia, polyethylene glycol (MiraLax®), Dulcolax®, Correctol® and Senokot®, anticholinergics or antispasmodics such as dicyclomine (Bentyl®), Singulair®, beta-2 agonists such as albuterol (Ventolin® HFA, Proventil® HFA), levalbuterol (Xopenex®), metaproterenol (Alupent®), pirbuterol acetate (Maxair®), terbutaline sulfate (Brethaire®), salmeterol xinafoate (Serevent®) and formoterol (Foradil®), anticholinergic agents such as ipratropium bromide (Atrovent®) and tiotropium (Spiriva®), inhaled corticosteroids such as beclomethasone dipropionate (Beclovent®, Qvar®, and Vanceril®), triamcinolone acetonide (Azmacort®), mometasone (Asthmanex®), budesonide (Pulmocort®), and flunisolide (Aerobid®), Afviar®, Symbicort®, Dulera®, cromolyn sodium (Intal®), methylxanthines such as theophylline (Theo-Dur®, Theolair®, Slo-bid®, Uniphyl®, Theo-24®) and aminophylline, IgE antibodies such as omalizumab (Xolair®), nucleoside reverse transcriptase inhibitors such as zidovudine (Retrovir®), abacavir (Ziagen®), abacavir/lamivudine (Epzicom®), abacavir/lamivudine/zidovudine (Trizivir®), didanosine (Videx®), emtricitabine (Emtriva®), lamivudine (Epivir®), lamivudine/zidovudine (Combivir®), stavudine (Zerit®), and zalcitabine (Hivid®), non-nucleoside reverse transcriptase inhibitors such as delavirdine (Rescriptor®), efavirenz (Sustiva®), nevairapine (Viramune®) and etravirine (Intelence®), nucleotide reverse transcriptase inhibitors such as tenofovir (Viread®), protease inhibitors such as amprenavir (Agenerase®), atazanavir (Reyataz®), darunavir (Prezista®), fosamprenavir (Lexiva®), indinavir (Crixivan®), lopinavir and ritonavir (Kaletra®), nelfinavir (Viracept®), ritonavir (Norvir®), saquinavir (Fortovase® or Invirase®), and tipranavir (Aptivus®), entry inhibitors such as enfuvirtide (Fuzeon®) and maraviroc (Selzentry®), integrase inhibitors such as raltegravir (Isentress®), doxorubicin (Hydrodaunorubicin®), vincristine (Oncovin®), bortezomib (Velcade®), and dexamethasone (Decadron ®) in combination with lenalidomide (Revlimid ®), anti-IL36 agents such as BI655130, Dihydroorotate dehydrogenase inhibitors such as IMU-838, anti-OX40 agents such as KHK-4083, microbiome agents such as RBX2660, SER-287, Narrow spectrum kinase inhibitors such as TOP-1288, anti-CD40 agents such as BI-655064 and FFP-104, guanylate cyclase agonists such as dolcanatide, sphingosine kinase inhibitors such as opaganib, anti-IL-12/IL-23 agents such as AK-101, Ubiquitin protein ligase complex inhibitors such as BBT- 401, sphingosine receptors modulators such as BMS-986166, P38MAPK/PDE4 inhibitors such as CBS-3595, CCR9 antagonists such as CCX-507, FimH antagonists such as EB-8018, HIF-PH inhibitors such as FG-6874, HIF-1$\alpha$ stabilizer such as GB-004, MAP3K8 protein inhibitors such as GS-4875, LAG-3 antibdies such as GSK-2831781, RIP2 kinase inhibitors such as GSK-2983559, Farnesoid X receptor agonist such as MET-409, CCK2 antagonists such as PNB-001, IL-23 Receptor antagonists such as PTG-200, Purinergic P2X7 receptor antagonists such as SGM-1019, PDE4 inhibiotrs such as Apremilast, ICAM-1 inhibitors such as alicaforsen sodium, Anti- IL23 agents such as guselkumab, brazikumab and mirkizumab, ant-IL-15 agents such as AMG-714, TYK-2 inhibitors such as BMS-986165, NK Cells activators such as CNDO-201, RIP-1 kinase inhibitors such as GSK-2982772, anti-NKGD2 agents such as JNJ-4500, CXCL-10 antibodies such as JT-02, IL-22 receptor agonists such as RG-7880, GATA-3 antagonists such as SB-012 and Colony-stimulating factor-1 receptor inhibitors such as edicotinib or any combination(s) thereof.

## Diseases caused by viruses

An ASD as defined in the present invention or a pharmaceutical composition as defined in the present invention, may be useful in the treatment or prevention of various diseases caused by viruses, in particular by retroviruses and more particularly by HIV, and more particularly for use for lowering viral load in a patient infected by a virus, in particular HIV, or a virus-related condition, with a long-lasting effect and absence of resistance.

Examples of viruses which are considered by the invention include enveloped and naked viruses, which includes DNA viruses, RNA viruses and retroviruses, which includes dsDNA viruses, ssDNA viruses, dsRNA viruses, (+)ssRNA viruses, (-)ssRNA viruses, ssRNA-RT viruses and dsDNA-RT viruses.

Viruses which are more particularly considered are RNA viruses and retroviruses, including lentiviruses, and preferably HIV. Accordingly, virus-related conditions which are more particularly considered are associated with a RNA virus or a retrovirus, and preferably HIV. HIV may include HIV-I, HIV-2 and all subtypes thereof, which includes HIV-I strains belonging to the HIV-I B subtype, HIV-I C subtype, and HIV-I recombinants. Examples include HIV-I strains selected from Ad8, AdaM, Isolate B, Isolate C, CRF01, CRF02 and CRF06. According to a preferred embodiment, the virus-related condition is AIDS.

Three subfamilies can be distinguished within the retroviral family: the oncoviruses, the lentiviruses and the spumaviruses. HIV pertains to the lentiviruses.

According to a particular embodiment, the retroviruses include HIV virus (HIV1 and HIV2), visna/maedi virus or MVV/visna, equine infectious anemia virus or EIAV, caprine arthritis encephalitis virus or CAEV, simian immunodeficiency virus or SIV, avian leukemia virus or ALV, murine leukemia virus also called Moloney virus or MULV, Abelson leukemia virus, murine mammary tumor virus, Mason-Pfizer monkey virus or MPMV, feline leukemia virus or FELV, human leukemia viruses HTLV-I, human leukemia viruses HTLV-II, simian leukemia virus or STLV, bovine leukemia virus or BLV, primate type D oncoviruses, type B oncoviruses, Rous sarcoma virus or RSV, simian foamy virus or SFV or chimpanzee simian virus, human foamy virus, and feline immunodeficiency virus, the human foamy virus or HFV, bovine syncytial virus or BSV, feline syncytial virus FSV, the feline immunodeficiency virus, avian leukosis virus, Walleye dermal sarcoma virus, T-cell lymphoma, acute ATL, lymphomatous ATL, chronic ATL, smoldering ATL, neurologic diseases, Tropical spastic paraparesis or HTLV-associated myelopathy, inflammatory and autoimmune diseases such as uveitis, dermatitis, pneumonitis, rheumatoid arthritis, and polymyositis hematologic and dermatologic diseases, lung diseases, brain diseases, and/or immunodeficiency.

As described herein, the term oncovirus can include Alpharetrovirus (for example, avian leukosis virus and Rous sarcoma virus); Betaretrovirus (for example, mouse mammary tumor virus); Gammaretrovirus (for example, murine leukemia virus and feline leukemia virus); Deltaretrovirus (for example bovine leukemia virus and human T-lymphotropic virus); and Epsilonretrovirus (for example, Walleye dermal sarcoma virus).

More generally, the retroviruses described herein may be, for example, visna/maedi virus or MVV/visna, equine infectious anemia virus or EIAV, caprine arthritis encephalitis virus or CAEV, simian immunodeficiency virus or SIV, avian leukemia virus or ALV, murine leukemia virus also called Moloney virus or MULV, Abelson leukemia virus, murine mammary tumor virus, Mason-Pfizer monkey virus or MPMV, feline leukemia virus or FELV, human leukemia viruses HTLV-I, human leukemia viruses HTLV-II, simian leukemia virus or STLV, the bovine leukemia virus or BLV, primate type D oncoviruses, type B oncoviruses, Rous sarcoma virus or RSV, and/or simian foamy virus or SFV or chimpanzee simian virus, human foamy virus, and feline immunodeficiency virus, the human foamy virus (or HFV), bovine syncytial virus (or BSV), feline syncytial virus (FSV) and the feline immunodeficiency virus.

More particularly, HTLV-I causes T-cell lymphoma (ATL for Adult T-cell leukemia/lymphoma, including the different forms of ATL such as acute ATL, lymphomatous ATL, chronic ATL and smoldering ATL), neurologic disease, Tropical spastic paraparesis (TSP) (also known as HTLV-associated myelopathy (HAM) or chronic progressive myelopathy), and diverse inflammatory and autoimmune diseases such as uveitis, dermatitis, pneumonitis, rheumatoid arthritis, and; HTLV-II may play a role in certain neurologic, hematologic and dermatologic diseases; HIV (HIV1 and HIV2) causes AIDS; the visna virus causes lung and brain diseases in sheep; the feline immunodeficiency virus causes immunodeficiency in cat; Rous sarcoma virus and mouse mammary tumor virus causes tumor growth and cancer.

The invention also relates to a method for treating and/or preventing diseases caused by viruses, in particular by retroviruses and more particularly by HIV, and which comprises a step of administering an ASD as defined in the present invention or a pharmaceutical composition as defined in the present invention to a patient in need thereof.

Additionally, the present invention has for purpose to lower a viral load in a patient infected by a virus, in particular HIV, or a virus-related condition, with a long-lasting effect and absence of resistance, by using the an ASD as defined in the present invention or the pharmaceutical composition as defined in the present invention.

In one embodiment, the present invention concerns an ASD as defined in the present invention or a pharmaceutical composition as defined in the present invention, for use for treating or preventing a retroviral infection or a retrovirus-related condition, in particular a HIV infection or a HIV-related condition in a patient, for which an ineffectiveness or a decline in a prior anti-retroviral treatment effectiveness has been stated.

In another embodiment, the present invention relates to an ASD as defined in the present invention or a pharmaceutical composition as defined in the present invention, for use for treating or preventing a retroviral infection or a retrovirus-related condition, in particular a HIV infection or a HIV-related condition in a patient, wherein the patient is infected by a drug-resistant viral strain, and more particularly by a drug-resistant HIV strain.

Furthermore, the invention further relates to new doses and regimens of said ASD as defined in the present invention and use in the treatment or prevention of viral infection, and in particular HIV, or a virus-related condition, more particularly where the use maintains a low viral load after treatment termination. Thus, according to one embodiment, the invention relates to an ASD as defined in the present invention or a pharmaceutical composition as defined in the present invention for use for treating or preventing of a virus infection or virus-related condition in a patient, in particular a HIV infection or a HIV-related condition, wherein: a low or undetectable viral load is maintained; and/or a CD4+ cell count is stable or increased; after treatment termination.

According to another embodiment, the invention relates to an ASD as defined in the present invention or a pharmaceutical composition as defined in the present invention, for use in the treatment or prevention of a virus infection or virus-related condition in patient, in particular a HIV infection or a HIV-related condition, for which an ineffectiveness in prior anti-retroviral treatment, or a decline in a prior anti-viral, or anti-retroviral, treatment effectiveness has been stated.

According to still another embodiment, the invention relates to an ASD as defined in the present invention or a pharmaceutical composition as defined in the present invention, for use in the treatment or prevention of a virus infection or virus-related condition in patient, in particular a HIV infection or a HIV-related condition, wherein the patient is infected by a drug-resistant strain.

In the framework of the present invention, the ASD as defined in the present invention may be administered in combination with another anti-retroviral agent. According to one embodiment, an ART (Antiretroviral Therapy) or HAART (Highly Active Antiretroviral Therapy) may be implemented using one or more of the following antiretroviral compounds:

(i) nucleoside/nucleotide reverse transcriptase inhibitors also called nucleoside analogs, such as abacavir, emtricitabine, and tenofovir;
(ii) non-nucleoside reverse transcriptase inhibitors (NNRTIs), such as efavirenz, etravirine, and nevirapine;
(iii) protease inhibitors (PIs), such as atazanavir, darunavir, and ritonavir;
(iv) entry inhibitors, such as enfuvirtide and maraviroc;
(v) integrase inhibitors, such as dolutegravir and raltegravir.

Other examples of anti-retroviral agents include, in a non-limitative manner: Zidovudine, Lamivudine, Emtricitabine, Didanosine, Stavudine, Abacavir, Zalcitabine, Racivir, Amdoxovir, Apricitabine, Elvucitabine, Efavirenz, Nevirapine, Etravirine, Delavirdine, Rilpvirine, Tenofovir, Fosalvudine, Amprenavir, Tipranavir, Indinavir, Saquinavir, Fosamprenavir, Ritonavir, Darunavir, Atazanavir, Nelfinavir, Lopinavir, Raltegravir, Elvitegravir, Dolutegravir, Enfuvirtide, Maraviroc, Vicriviroc, and combinations thereof.

In some embodiments, an ASD according to the present invention may be administered in combination with one or more additional therapeutic agents selected from nucleoside reverse transcriptase inhibitors such as zidovudine (Retrovir®), abacavir (Ziagen®), abacavir/lamivudine (Epzicom®), abacavir/lamivudine/zidovudine (Trizivir®), didanosine (Videx®), emtricitabine (Emtriva®), lamivudine (Epivir®), lamivudine/zidovudine (Combivir®), stavudine (Zerit®), and zalcitabine (Hivid®), non-nucleoside reverse transcriptase inhibitors such as delavirdine (Rescriptor®), efavirenz (Sustiva®), nevairapine (Viramune®) and etravirine (Intelence®), nucleotide reverse transcriptase inhibitors such as tenofovir (Viread®), protease inhibitors such as amprenavir (Agenerase®), atazanavir (Reyataz®), darunavir (Prezista®), fosamprenavir (Lexiva®), indinavir (Crixivan®), lopinavir and ritonavir (Kaletra®), nelfinavir (Viracept®), ritonavir (Norvir®), saquinavir (Fortovase® or Invirase®), and tipranavir (Aptivus®), entry inhibitors such as enfuvirtide (Fuzeon®) and maraviroc (Selzentry®), integrase inhibitors such as raltegravir (Isentress®), and combinations thereof.

### Cancers

An ASD as defined in the present invention or a pharmaceutical composition as defined in the present invention, may be useful in the treatment or prevention of various cancers.

As used herein the term "cancer", and unless stated otherwise, may relate to any disorder associated with abnormal cell growth, which thus includes malignant tumors and benign tumors, metastatic tumors and non-metastatic tumors, solid tumors and non-solid tumors, such as Blood-Related Cancers which may thus include Leukaemia, Lymphoma and Myeloma; it may also relate to Central Nervous System (CNS) cancers and non-CNS cancers. Unless stated otherwise, the term *"cancer"* also encompasses juvenile and non-juvenile cancers, Recurrent and Non-Recurrent cancers as well as cancer relapses.

Among cancers, the following may be cited: Blood-Related Cancer, pancreatic cancer, urological cancer, bladder cancer, colorectal cancer, colon cancer, breast cancer, prostate cancer, renal cancer, hepatocellular cancer, thyroid cancer, gall bladder cancer, lung cancer (e.g. non-small cell lung cancer, small-cell lung cancer), ovarian cancer, cervical cancer, gastric cancer, endometrial cancer, oesophageal cancer, head and neck cancer, melanoma, neuroendocrine cancer, CNS cancer, brain tumors (e.g., glioma, anaplastic oligodendroglioma, adult glioblastoma multiforme, and adult anaplastic astrocytoma), bone cancer, soft tissue sarcoma, retinoblastomas, neuroblastomas, peritoneal effusions, malignant pleural effusions, mesotheliomas, Wilms tumors, trophoblastic neoplasms, hemangiopericytomas, Kaposi's sarcomas, myxoid carcinoma, round cell carcinoma, squamous cell carcinomas, oesophageal squamous cell carcinomas, oral carcinomas, cancers of the adrenal cortex, or ACTH-producing tumors.

According to one embodiment, the following cancers may be cited: head and neck cancer, stomach cancer, breast cancer, basal and squamous skin cell cancer, liver cancer, kidney cancer, brain cancer, lung cancer, pancreatic cancer, eye cancer, gastrointestinal cancer, colorectal cancer, oesophageal cancer, colorectal cancer, bladder cancer, gall bladder cancer, thyroid cancer, melanoma, uterine/cervical cancer, ovarian, cancer, bone cancer and renal cancer.

According to another embodiment, the cancer may be selected from head and neck cancer, Head and Neck Squamous Cell Carcinoma, Neck Squamous Cell Carcinoma, Acute Lymphocytic Leukemia (ALL) in Adults or children, Acute Myeloid Leukemia (AML) in adults or children, Acute Lymphoblastic Leukemia, Adrenal Cancer, Anal Cancer, Astrocytic Glioma, Astrocytoma (grade I, II, III, or IV), B- or NK/T-cell lymphomas, Basal and Squamous Skin Cell Cancer, Bile Duct Cancer, Bladder Cancer, Bone Cancer, brain cancer, Brain and Spinal Cord Tumors in Adults, Brain and Spinal Cord Tumors in Children, Anaplastic astrocytomas, Breast cancer, Gastrointestitnal cancer, Breast Cancer in Women, Breast Cancer in Young Women, Breast Cancer in Men, Recurrent Breast Cancer, Hereditary Breast Cancer, HER2 positive Breast Cancer, Breast Cancer associated with lymph node metastatis, ER-alpha positive Breast Cancer, Cancer in Adolescents, Cancer in Children, Cancer in Young Adults, Cancer of Unknown Primary, Castleman Disease, Cervical Cancer, Cervical Intraepithelial Neoplasia, Cholangiocarcinoma, Chronic Lymphocytic Leukemia (CLL), Chronic Myeloid Leukemia (CML), Chronic Myelomonocytic Leukemia (CMML), Colorectal Cancer, colorectal adenoma, Cutaneous Squamous Cell Carcinoma, Endometrial Cancer, Epithelial Ovarian Cancer, Epithelial Ovarian Cancer associated with metastasis, esophageal cancer, esophagus Squamous Cell Carcinoma, Ewing sarcoma, Ewing Family of Tumors, Lymphoblastic leukaemia (ALL), Eye Cancer, such as Ocular Melanoma and Lymphoma, Gallbladder Cancer, Gastric Cancer, Gastrointestinal Carcinoid Tumors, Gastrointestinal Stromal Tumor (GIST), Gestational Trophoblastic Disease, Glioblastoma, Glioblastoma multiforme (GBM), Hairy cell leukemia, Glioma, High-grade glioma, Hepatocellular carcinoma, Intrahepatic cholangiocarcinoma, Invasive Breast Ductal Carcinoma, Hodgkin Lymphoma, Kaposi Sarcoma, Kidney Cancer, Laryngeal and Hypopharyngeal Cancer, Leiomyosarcoma, Leukemia, Leukemia in Children, Liver Cancer, Lung Cancer, Lung Carcinoid Tumor, Lymphoma, Lymphoma of the Skin, Malignant Mesothelioma, Mantle cell lymphoma, Medulloblastoma, Melanoma Skin Cancer, malignant melanoma, Meningioma, Merkel Cell Skin Cancer, Multiple Myeloma, Multiple Myeloma with Osteonecrosis of the Jaw, Myelodysplastic Syndrome, Nasal Cavity and Paranasal Sinuses Cancer, Nasopharyngeal Cancer, recurrent or metastatic Nasopharyngeal carcinoma, Neuroblastoma, Neuroglioma, Non-Hodgkin Lymphoma, Non-Hodgkin Lymphoma in Children, Non-Small Cell Lung Cancer, Gefitinib-resistant non-small cell lung cancer, Oral cancer, Oral Cavity and Oropharyngeal Cancer, Osteosarcoma, Pulmonary Metastatic Osteosarcoma, Ovarian Cancer, Pancreatic Cancer, thyroid carcinoma, Papillary Thyroid Carcinoma, Pediatric Spinal Ependymoma, Penile Cancer, Pituitary Tumors, Pituitary Adenoma, Proneural tumors, Prostate Cancer, Retinoblastoma, Rhabdomyosarcoma, Salivary Gland Cancer, Skin Cancer, Small Cell Lung Cancer, Small Intestine Cancer, Soft Tissue Sarcoma, Squamous Cell Carcinoma of the Tongue, Stomach Cancer, Testicular Cancer, Thymus Cancer, Thyroid Cancer, Uterine Sarcoma, Vaginal Cancer, Vulvar Cancer, Renal cancer, Retinoblastoma, Waldenstrom Macroglobulinemia and Wilms Tumor.

According to another embodiment, the cancer may be selected from a Head and Neck Squamous Cell Carcinoma, Neck Squamous Cell Carcinoma, Acute Lymphocytic Leukemia (ALL) in Adults or children, Acute Myeloid Leukemia (AML) in adults or children, Acute Lymphoblastic Leukemia, Adrenal Cancer, Anal Cancer, Astrocytic Glioma, Astrocytoma (grade I, II, III, or IV), B- or NK/T-cell lymphomas, Basal and Squamous Skin Cell Cancer, Bile Duct Cancer, Bone Cancer, brain cancer, Brain and Spinal Cord Tumors in Adults, Brain and Spinal Cord Tumors in Children, Anaplastic astrocytomas, Gastrointestitnal cancer, Breast Cancer in Women, Breast Cancer in Young Women, Breast Cancer in Men, Recurrent Breast Cancer, Hereditary Breast Cancer, HER2 positive Breast Cancer, Breast Cancer associated with lymph node metastatis, ER-alpha positive Breast Cancer, Cancer in Adolescents, Cancer in Children, Cancer in Young Adults, Cancer of Unknown Primary, Castleman Disease, Cervical Intraepithelial Neoplasia, Cholangiocarcinoma, Chronic Lymphocytic Leukemia (CLL), Chronic Myeloid Leukemia (CML), Chronic Myelomonocytic Leukemia (CMML), colorectal adenoma, Cutaneous Squamous Cell Carcinoma, Endometrial Cancer, Epithelial Ovarian Cancer, Epithelial Ovarian Cancer associated with metastasis, esophagus Squamous Cell Carcinoma, Ewing sarcoma, Ewing Family of Tumors, Lymphoblastic leukaemia (ALL), Eye Cancer, such as Ocular Melanoma and Lymphoma, Gastric Cancer, Gastrointestinal Carcinoid Tumors, Gastrointestinal Stromal Tumor (GIST), Gestational Trophoblastic Disease, Glioblastoma, Glioblastoma multiforme (GBM), Hairy cell leukemia, Glioma, High-grade glioma, Hepatocellular carcinoma, Intrahepatic cholangiocarcinoma, Invasive Breast Ductal Carcinoma, Hodgkin Lymphoma, Kaposi Sarcoma, Laryngeal and Hypopharyngeal Cancer, Leiomyosarcoma, Leukemia, Leukemia in Children, Lung Carcinoid Tumor, Lymphoma, Lymphoma of the Skin, Malignant Mesothelioma, Mantle cell lymphoma, Medulloblastoma, malignant melanoma, Meningioma, Merkel Cell Skin Cancer, Multiple Myeloma, Multiple Myeloma with Osteonecrosis of the Jaw, Myelodysplastic Syndrome, Nasal Cavity and Paranasal Sinuses Cancer, Nasopharyngeal Cancer, recurrent or metastatic Nasopharyngeal carcinoma, Neuroblastoma, Neuroglioma, Non-Hodgkin Lymphoma, Non-Hodgkin Lymphoma in Children, Gefitinib-resistant non-small cell lung cancer, Oral cancer, Oral Cavity and Oropharyngeal Cancer, Osteosarcoma, Pulmonary Metastatic Osteosarcoma, thyroid carcinoma, Papillary Thyroid Carcinoma, Pediatric Spinal Ependymoma, Penile Cancer, Pituitary Tumors, Pituitary Adenoma, Proneural tumors, Retinoblastoma, Rhabdomyosarcoma, Salivary Gland Cancer, Skin Cancer, Small Cell Lung Cancer, Small Intestine Cancer, Soft Tissue Sarcoma, Squamous Cell Carcinoma of the Tongue, Testicular Cancer, Thymus Cancer, Uterine Sarcoma, Vaginal Cancer, Vulvar Cancer, Renal cancer, Retinoblastoma, Waldenstrom Macroglobulinemia and Wilms Tumor.

According to a further embodiment, the cancer may be selected from head and neck cancer, Head and Neck Squamous Cell Carcinoma, Neck Squamous Cell Carcinoma, Malignant melanoma, stomach cancer, Breast cancer, Breast cancer in Women, Breast Cancer in Young Women, basal and squamous skin cell cancer, liver cancer, brain cancer, Anaplastic astrocytomas, lung cancer, Non-Small Cell Lung Cancer, Gefitinib-resistant non-small cell lung cancer, Oral cancer, eye cancer, Gastric Cancer, gastrointestinal cancer, Astrocytic Glioma, Astrocytoma (grade I, II, III, or IV), colorectal cancer, colorectal adenoma, Cutaneous Squamous Cell Carcinoma, bladder cancer, bone cancer, Recurrent Breast Cancer, Hereditary Breast Cancer, HER2 positive Breast Cancer, Breast Cancer associated with lymph node metastatis, ER-alpha positive Breast Cancer, renal cancer, Cervical Intraepithelial Neoplasia, Cholangiocarcinoma, Leiomyosarcoma, Chronic Lymphocytic Leukemia (CLL), Chronic Myeloid Leukemia (CML), Chronic Myelomonocytic Leukemia (CMML), Acute Myeloid Leukemia (AML) in adults or children, Acute Lymphoblastic Leukemia,, B- or NK/T-cell lymphomas, cervical cancer, Glioblastoma, Glioblastoma multiforme (GBM), Hairy cell leukemia, Glioma, High-grade glioma, Hepatocellular carcinoma, Intrahepatic cholangiocarcinoma, Invasive Breast Ductal Carcinoma, kidney cancer, Endometrial cancer, ovarian cancer, Epithelial Ovarian Cancer, Epithelial Ovarian Cancer associated with metastasis, esophageal cancer, esophageal Squamous Cell Carcinoma, Ewing sarcoma, Lymphoblastic leukaemia (ALL), Mantle cell lymphoma, Medulloblastoma, Lymphoma, Myelodysplastic syndrome, Meningioma, Multiple Myeloma (MM), Multiple Myeloma with Osteonecrosis of the Jaw, Nasopharyngeal Cancer, recurrent or metastatic Nasopharyngeal carcinoma, Neuroblastoma, Neuroglioma, Papillary Thyroid Carcinoma, Pediatric Spinal Ependymoma, Osteosarcoma, Pulmonary Metastatic Osteosarcoma, pancreatic cancer, thyroid carcinoma, sarcoma, pituitary tumors, Pituitary Adenoma, Proneural tumors, Squamous Cell Carcinoma of the Tongue, Mesothelioma, Retinoblastoma and prostate cancer.

According to a further embodiment, the cancer may be selected from Head and Neck cancer, Head and Neck Squamous Cell Carcinoma, Neck Squamous Cell Carcinoma, malignant melanoma, Astrocytic Glioma, Glioma, stomach cancer, Breast cancer, Cholangiocarcinoma, recurrent or metastatic Nasopharyngeal carcinoma, basal and squamous skin cell cancer, liver cancer, brain cancer, Anaplastic astrocytomas, lung cancer, Non-Small Cell Lung Cancer, Gefitinib-resistant non-small cell lung cancer, Oral cancer, Glioblastoma, osteosarcoma, Pulmonary Metastatic Osteosarcoma, pancreatic cancer, eye cancer, gastrointestinal cancer, colorectal cancer, colorectal adenoma, Cutaneous Squamous Cell Carcinoma, Endometrial cancer, Epithelial Ovarian Cancer, esophageal cancer, Ewing sarcoma, gastric cancer, Hepatocellular carcinoma, HER2 positive Breast Cancer, bladder cancer, bone cancer, prostate cancer, Retinoblastoma and renal cancer.

According to a further embodiment, the cancer may be selected from Anaplastic astrocytomas, Astrocytic gliomas, Bladder cancer, Breast cancer, Cholangiocarcinoma, Colorectal cancer, Colorectal adenoma, Cutaneous squamous cell carcinoma, Endometrial cancer, Epithelial ovarian cancer, Esophageal cancer, Ewing sarcoma, Gastric cancer, Gefitinib-resistant non-small cell lung cancer, Glioblastoma, Glioma, Hepatocellular carcinoma, HER2 positive breast cancer, Head and Neck Squamous Cell Carcinoma, Malignant melanoma, Nasopharyngeal carcinoma (recurrence or metastasis), Neck squamous cell carcinoma, Non-small cell lung cancer, Oral cancer, Osteosarcoma, Osteosarcoma (pulmonary metastasis), Prostate cancer and retinoblastoma

According to a further embodiment, the cancer may be selected from anal cancer, bile duct cancer, gastrointestinal cancer, Cholangiocarcinoma, colorectal cancer, colorectal adenoma, esophageal cancer, Esophagus Squamous Cell Carcinoma ,gastric cancer, Gastrointestinal Carcinoid Tumors, Gastrointestinal Stromal Tumor (GIST), Hepatocellular carcinoma, Intrahepatic cholangiocarcinoma, liver cancer, lung cancer, Lung Carcinoid Tumor, non-Small Cell Lung Cancer, Gefitinib-resistant non-small cell lung cancer, Pulmonary Metastatic Osteosarcoma, stomach cancer, pancreatic cancer, Small Cell Lung Cancer, and Small Intestine Cancer

According to one embodiment, the patient does not present clinically detectable metastases, in particular said patient has a pre-cancerous condition, an early stage cancer or a non-metastatic cancer, or said patient presents clinically detectable metastases and said ASD as defined in the present invention does not target directly the invasion of metastases.

In view of the above, the invention relates to an ASD as defined in the present invention or a pharmaceutical composition as defined in the present invention for use in the treatment and/or prevention of cancer, such as the here above listed cancers, and dysplasia.

Thus, the invention also relates to the use of an ASD as defined in the present invention or a pharmaceutical composition as defined in the present invention for treating and/or preventing cancer, such as the here above listed cancers, and dysplasia.

The invention also relates to the use of an ASD as defined in the present invention for the preparation of a composition, such as a medicament, for treating and/or preventing of cancer, such as the here above listed cancers, and dysplasia.

The invention also relates to a method of preventing, inhibiting or treating cancer or dysplasia, which comprises at least one step consisting in administering to a patient suffering therefrom an effective amount of an ASD as defined in the present invention or a pharmaceutical composition as defined in the present invention.

In some embodiments, the invention relates to a method of the present invention or an ASD as defined in the present invention or a pharmaceutical composition as defined in the present invention for use as defined above, for treating and/or preventing cancer or dysplasia, wherein a presence and/or expression level of miR-124 in a blood and/or tissue sample of the patient, is measured prior to and/or during the use.

In some embodiments, the invention relates to a method of the present invention or an ASD as defined in the present invention or a pharmaceutical composition as defined in the present invention for use as defined above, for treating and/or preventing cancer or dysplasia, wherein a presence and/or expression level of miR-124 in a blood and/or tissue sample is measured to guide dose or monitor response to the treatment.

In some embodiments, the invention relates to a method of the present invention or an ASD as defined in the present invention or a pharmaceutical composition as defined in the present invention for use as defined above, for treating and/or preventing cancer or dysplasia, wherein the level of an ASD as defined in the present invention, in a blood, plasma, tissue, saliva, and/or serum sample of the patient is measured during the use.

In some embodiments, the invention relates to a method of the present invention or an ASD as defined in the present invention or a pharmaceutical composition as defined in the present invention for use as defined above, for treating and/or preventing cancer or dysplasia, which is used in combination with another anti-tumoral agent.

In some embodiments, the invention relates to a method of the present invention or an ASD as defined in the present invention or a pharmaceutical composition as defined in the present invention for use as defined above, for treating and/or preventing cancer or dysplasia, which is used in combination with another therapy selected from chemotherapy, immunotherapy, radiotherapy, surgery, ultrasounds, monoclonal antibodies, and cancer vaccines.

Among other anticancer drug, the following may be cited:

- Androgen receptor inhibitors, such as enzalutamide (Xtandi®, Astellas/Medivation), abiraterone (Zytiga®, Centocor/Ortho), antagonist of gonadotropin-releasing hormone (GnRH) receptor such as degaralix, Firmagon®, Ferring Pharmaceuticals)
- **Antiapoptotics,** such as venetoclax (Venclexta®, AbbVie/Genentech), blinatumomab (Blincyto®, Amgen), navitoclax (ABT-263, Abbott);
- **Antiproliferative and Antimitotic agents,** such as vinca alkaloids (which include vinblastine, vincristine);
- **Antibiotics** such as dactinomycin, daunorubicin, doxorubicin, idarubicin, anthracyclines, mitoxantrone, bleomycins, plicamycin (mithramycin), and mitomycin;
- **L-asparaginase;**
- **Antiplatelet agents;**
- **Antiproliferative/antimitotic alkylating agents** such as nitrogen mustards cyclophosphamide and analogs (which include melphalan, chlorambucil, hexamethylmelamine, and thiotepa), alkyl nitrosoureas (which include carmustine) and analogs, streptozocin, and triazenes (which include dacarbazine);
- **Antiproliferative/antimitotic antimetabolites** such as folic acid analogs (which include methotrexate), aromatase inhibitors; antiestrogens; topoisomerase I inhibitors; topoisomerase II inhibitors; microtubule active compounds; alkylating compounds; histone deacetylase inhibitors; compounds which induce cell differentiation processes; cyclooxygenase inhibitors; MMP inhibitors; mTOR inhibitors; antineoplastic antimetabolites; platin compounds; compounds targeting/decreasing a protein or lipid kinase activity and further anti-angiogenic compounds; compounds which target, decrease or inhibit the activity of a protein or lipid phosphatase; gonadorelin agonists; antiandrogens; methionine aminopeptidase inhibitors; matrix metalloproteinase inhibitors; bisphosphonates; biological response modifiers; antiproliferative antibodies; heparanase inhibitors; inhibitors of Ras oncogenic isoforms; telomerase inhibitors; proteasome inhibitors; compounds used in the treatment of hematologic malignancies; compounds which target, decrease or inhibit the activity of Flt-3; Hsp90 inhibitors such as 17-AAG (17-allylaminogeldanamycin, NSC330507), 17-DMAG (17-dimethylaminoethylamino-17-demethoxy-geldanamycin, NSC707545), IPI-504, CNF1010, CNF2024, CNF1010 from Conforma Therapeutics; temozolomide (Temodal®); kinesin spindle protein inhibitors, such as SB715992 or SB743921 from GlaxoSmithKline, or pentamidine/chlorpromazine from CombinatoRx; MEK inhibitors such as ARRY142886 from Array BioPharma, $AZd_6244$ from AstraZeneca, PD181461 from Pfizer and leucovorin;
- **Antimigratory agents;**
- **Angiogenesis inhibitors,** such as TNP-470;
- **Aromatase inhibitors,** such as letrozole and anastrozole, exemestane;

- **Angiotensin**
- **Anti-sense oligonucleotides,** such as antisense nucleic acids directed toward miR-124;
- **Anticoagulants,** such as heparin, synthetic heparin salts, and other inhibitors of thrombin;
- **Arginine inhibitors,** such as AEB1102 (pegylated recombinant arginase, Aeglea Biotherapeutics) and CB-1158 (Calithera Biosciences);
- **Bone resorption inhibitors,** such as Denosumab (Xgeva®, Amgen), bisphosphonates such as zoledronic acid (Zometa®, Novartis);
- **CC chemokine receptor 4 (CCR4) inhibitors,** such as mogamulizumab (Poteligeo®, Kyowa Hakko Kirin, Japan);
- **CDK inhibitors,** such as CDK4/CDK6 inhibitors, such as palbociclib (Ibrance®, Pfizer); ribociclib (Kisqali®, Novartis); abemaciclib (Ly2835219, Eli Lilly); and trilaciclib (G1T28, G1 Therapeutics) ;
- **Cell cycle inhibitors and differentiation inducers,** such as as tretinoin;
- **Corticosteroids,** such as cortisone, dexamethasone, hydrocortisone, methylprednisolone, prednisone, and prednisolone;
- **DNA damaging agents** such as actinomycin, amsacrine, busulfan, carboplatin, chlorambucil, cisplatin, cyclophosphamide (CYTOXAN®), dactinomycin, daunorubicin, doxorubicin, epirubicin, iphosphamide, melphalan, merchlorethamine, mitomycin, mitoxantrone, nitrosourea, procarbazine, taxol, taxotere, teniposide, etoposide, and triethylenethiophosphoramide;
- **Fibrinolytic agents,** such as tissue plasminogen activator, streptokinase, urokinase, aspirin, dipyridamole, ticlopidine, and clopidogrel;
- **Folate antagonists;**
- **FLT3 receptor inhibitors,** such as enzalutamide, abiraterone, apalutamide, erlotinib, crizotinib, niraparib, olaparib, osimertinib, regorafenib, sunitinib, lestaurtinib, midostaurin, gilteritinib, semaxinib, linifanib, fostamatinib, pexidartinib, sorafenib, cabozantinib, ponatinib, ilorasertib, pacritinib, famitinib, pexidartinib, quizartinib;
- **Glutaminase inhibitors,** such as CD-839 (Calithera Biosciences);
- **Growth Factor Signal transduction kinase inhibitors;**
- **Growth factor Inhibitors,** such as vascular endothelial growth factor inhibitors and fibroblast growth factor inhibitors, such as olaratumab (Lartruvo®; Eli Lilly), cetuximab (Erbitux®, Eli Lilly); necitumumab (Portrazza®, Eli Lilly), panitumumab (Vectibix®, Amgen); and osimertinib (targeting activated EGFR, Tagrisso®, AstraZeneca);
- **Hedgehog pathway inhibitors,** such as sonidegib (Odomzo®, Sun Pharmaceuticals); and vismodegib (Erivedge®, Genentech);
- **Histone deacetylase (HDAC) inhibitors,** such as vorinostat (Zolinza®, Merck); romidepsin (Istodax®, Celgene); panobinostat (Farydak®, Novartis); belinostat (Beleodaq®, Spectrum Pharmaceuticals); entinostat (SNDX-275, Syndax Pharmaceuticals) (NCT00866333); and chidamide (Epidaza®, HBI-8000, Chipscreen Biosciences, China);
- **Hormones and analogs thereof,** such as estrogen, tamoxifen, goserelin, bicalutamide, and nilutamide);
- **Isocitrate dehydrogenase (IDH) inhibitors,** such as AG120 (Celgene; NCT02677922); AG221 (Celgene, NCT02677922; NCT02577406); BAY1436032 (Bayer, NCT02746081); IDH305 (Novartis, NCT02987010)
- **Isoflavones** such as genistein;
- **Immunosuppressives,** such as tacrolimus, sirolimus, azathioprine, and mycophenolate;
- **Inhibitors of p53 suppressor proteins,** such as ALRN-6924 (Aileron);
- **Inhibitors of transforming growth factor-beta (TGF-beta or TGF$\beta$),** such as NIS793 (Novartis), fresolimumab (GC1008; Sanofi-Genzyme), M7824 (Merck KgaA - formerly MSB0011459X);
- iNKT cell agonists asuch as ABX196 5Abivax)
- **mTOR inhibitors,** such as everolimus (Afinitor®, Novartis); temsirolimus (Torisel®, Pfizer); and sirolimus (Rapamune®, Pfizer) ;
- **Microtubule-inhibiting drugs,** such as taxanes (which include paclitaxel, docetaxel), vinblastin, nocodazole, epothilones, vinorelbine) (NAVELBINE®), and epipodophyllotoxins (etoposide, teniposide);
- **Nitric oxide donors;**
- **Nucleoside inhibitors,** such as trabectedin (guanidine alkylating agent, Yondelis®, Janssen Oncology), mechlorethamine (alkylating agent, Valchlor®, Aktelion Pharmaceuticals); vincristine (Oncovin®, Eli Lilly; Vincasar®, Teva Pharmaceuticals; Marqibo®, Talon Therapeutics); temozolomide (prodrug to alkylating agent 5-(3-methyltriazen-1-yl)-imidazole-4-carboxamide (MTIC) Temodar®, Merck); cytarabine injection (ara-C, antimetabolic cytidine analog, Pfizer); lomustine (alkylating agent, CeeNU®, Bristol-Myers Squibb; Gleostine®, NextSource Biotechnology); azacitidine (pyrimidine nucleoside analog of cytidine, Vidaza®, Celgene); omacetaxine mepesuccinate (cephalotaxine ester) (protein synthesis inhibitor, Synribo®; Teva Pharmaceuticals); asparaginase *Erwinia chrysanthemi* (enzyme for depletion of asparagine, Elspar®, Lundbeck; Erwinaze®, EUSA Pharma); eribulin mesylate (microtubule inhibitor, tubulin-based antimitotic, Halaven®, Eisai); cabazitaxel (microtubule inhibitor, tubulin-based antimitotic, Jevtana®, Sanofi-Aventis); capacetrine (thymidylate synthase inhibitor, Xeloda®, Genentech); bendamustine (bifunctional mechlorethamine derivative, believed to form interstrand DNA cross-links, Treanda®, Cephalon/Teva); ixabepilone (semi-synthetic analog of epothilone B, microtubule inhibitor, tubulin-based antimitotic, Ixempra®, Bristol-Myers Squibb); nelarabine (prodrug of deoxyguanosine analog, nucleoside metabolic inhibitor, Arranon®, Novartis); clorafabine (prodrug of ribonucleotide reductase inhibitor, competitive inhibitor of deoxycytidine, Clolar®, Sanofi-Aventis); and trifluridine and tipiracil (thymidine-based nucleoside analog and thymidine phosphorylase inhibitor, Lonsurf®, Taiho Oncology);

- **PI3K inhibitors,** such as idelalisib (Zydelig®, Gilead), alpelisib (BYL719, Novartis), taselisib (GDC-0032, Genentech/Roche); pictilisib (GDC-0941, Genentech/Roche); copanlisib (BAY806946, Bayer); duvelisib (formerly IPI-145, Infinity Pharmaceuticals); PQR309 (Piqur Therapeutics, Switzerland); and TGR1202 (formerly RP5230, TG Therapeutics);
- **Platinum coordination complexes** (such as cisplatin, oxiloplatin, carboplatin, nedaplatin, picoplatin, procarbazine, mitotane, satraplatin and aminoglutethimide;
- **Poly ADB ribose polymerase (PARP) inhibitor,** such as those selected from: olaparib (Lynparza®, AstraZeneca); rucaparib (Rubraca®, Clovis Oncology); niraparib (Zejula®, Tesaro); talazoparib (MDV3800/BMN 673/LT00673, Medivation/Pfizer/Biomarin); veliparib (ABT-888, AbbVie); and BGB-290 (BeiGene, Inc.) ;
- **Proteasome inhibitors,** such as everolimus (Afinitor®, Novartis); temsirolimus (Torisel®, Pfizer); and sirolimus (Rapamune®, Pfizer), bortezomib (Velcade®, Takeda); carfilzomib (Kyprolis®, Amgen); and ixazomib (Ninlaro®, Takeda);
- **Pyrimidine & Purine analogs,** such as floxuridine, capecitabine, and cytarabine;
- **Receptor blockers, Antisecretory agents,** such as breveldin;
- **Selective estrogen receptor modulator (SERM),** such as raloxifene (Evista®, Eli Lilly);
- **Therapeutic antibodies,** such as those selected from: anti-TNF antibodies, anti-VEGF antibodies, anti-EGFR antibodies, anti-PD-1 antibodies, anti-HER2 antibodies, anti-CD20 antibodies, anti-IL17 antibodies, and anti-CTLA4 antibodies, anti-PDL1, anti-CD25, anti-α4integrin, anti-IL6R, anti-C5, anti-IL1, anti-TPO, anti-IL12/23, anti-EPCAM/CD3, anti-CD30, anti-CD80/86, anti-anthrax, anti-CCR4, anti-CD6, anti-CD19, anti-α4β7, anti-IL6, anti-VEGFR-2, anti-SLAMF7, anti-GD2, anti-IL17A, anti-PCSK9, anti-IL5, anti-CD22, anti-IL4, anti-PDGFRα, anti-IL17RA and anti-TcdB, and such as those selected from: Abagovomab, Abatacept, Abciximab, Abituzumab, Abrilumab, Actoxumab, Adalimumab, Adecatumab, Aducanumab, Aflibercept, Afutuzymab, Alacizumab, Alefacept, Alemtuzumab, Alirocumab, Altumomab, Amatixumab, Anatumomab, Anetumab, Anifromumab, Anrukinzumab, Apolizumab, Arcitumomab, Ascrinvacumab, Aselizumab, Atezolizumab, Atinumab, Altizumab, Atorolimumab, Bapineuzumab, Basiliximab, Bavituximab, Bectumomab, Begelomab, Belatacept, Belimumab, Benralizumab, Bertilimumab, Besilesomab, Bevacizumab, Bezlotoxumab, Biciromab, Bimagrumab, Bimekizumab, Bivatuzumab, Blinatumomab, Blosozumab, Bococizumab, Brentuximab, Briakimumab, Brodalumab, Brolucizumab, Bronticizumab, Canakinumab, Cantuzumab, Caplacizumab, Capromab, Carlumab, Catumaxomab, Cedelizumab, Certolizumab, Cetixumab, Citatuzumab, Cixutumumab, Clazakizumab, Clenoliximab, Clivatuzumab, Codrituzumab, Coltuximab, Conatumumab, Concizumab, Crenezumab, Dacetuzumab, Daclizumab, Dalotuzumab, Dapirolizumab, Daratumumab, Dectrekumab, Demcizumab, Denintuzumab, Denosumab, Derlotixumab, Detumomab, Dinutuximab, Diridavumab, Dorlinomab, Drozitumab, Dupilumab, Durvalumab, Dusigitumab, Ecromeximab, Eculizumab, Edobacomab, Edrecolomab, Efalizumab, Efungumab, Eldelumab, Elgemtumab, Elotuzumab, Elsilimomab, Emactuzumab, Emibetuzumab, Enavatuzumab, Enfortumab, Enlimomab, Enoblituzumab, Enokizumab, Enoticumab, Ensituximab, Epitumomab, Epratuzomab, Erlizumab, Ertumaxomab, Etanercept, Etaracizumab, Etrolizumab, Evinacumab, Evolocumab, Exbivirumab, Fanolesomab, Faralimomab, Farletuzomab, Fasimumab, Felvizumab, Fezkimumab, Ficlatuzumab, Figitumumab, Firivumab, Flanvotumab, Fletikumab,Fontolizumab, Foralumab, Foravirumab, Fresolimumab, Fulramumab, Futuximab, Galiximab, Ganitumab, Gantenerumab, Gavilimomab, Gemtuzumab, Gevokizumab, Girentuximab, Glembatumumab, Golimumab, Gomiliximab, Guselkumab, Ibalizumab, Ibritumomab, Icrucumab, Idarucizumab, Igovomab, Imalumab, Imciromab, Imgatuzumab, Inclacumab, Indatuximab, Indusatumab, Infliximab, Intetumumab, Inolimomab, Inotuzumab, Ipilimumab, Iratumumab, Isatuximab, Itolizumab, Ixekizumab, Keliximab, Labetuzumab, Lambrolizumab, Lampalizumab, Lebrikizumab, Lemalesomab, Lenzilumab, Lerdelimumab, Lexatumumab, Libivirumab, Lifastuzumab, Ligelizumab, Lilotomab, Lintuzumab, Lirilumab, Lodelcizumab, Lokivetmab, Lorvotuzumab, Lucatumumab, Lulizumab, Lumiliximab, Lumretuzumab, Mapatumumab, Margetuximab, Maslimomab, Mavrilimumab, Matuzumab, Mepolizumab, Metelimumab, Milatuzumab, Minetumomab, Mirvetuximab, Mitumomab, Mogamulizumab, Morolimumab, Motavizumab, Moxetumomab, Muromonab-CD3, Nacolomab, Namilumab, Naptumomab, Narnatumab, Natalizumab, Nebacumab, Necitumumab, Nemolizumab, Nerelimomab, Nesvacumab, Nimotuzumab, Nivolumab, Nofetumomab, Obiltoxaximab, Obinutuzumab, Ocaratuzumab, Ocrelizumab, Odulimomab, Ofatumumab, Olaratumab, Olokizumab, Omalizumab, Onartuzumab, Ontuxizumab, Opicinumab, Oportuzumab, Oregovomab, Orticumab, Otelixizumab, Oltertuzumab, Oxelumab, Ozanezumab, Ozoralizumab, Pagibaximab, Palivizumab, Panitumumab, Pankomab, Panobacumab, Parsatuzumab, Pascolizumab, Pasotuxizumab, Pateclizumab, Patritumab, Pembrolizumab, Pemtumomab, Perakizumab, Pertuzumab, Pexelizumab, Pidilizumab, Pinatuzumab, Pintumomab, Polatuzumab, Ponezumab, Priliximab, Pritumumab, Quilizumab, Racotumomab, Radretumab, Rafivirumab, Ralpancizumab, Ramucirumab, Ranibizumab, Raxibacumab, Refanezumab, Regavirumab, Reslizumab, Rilonacept, Rilotumumab, Rinucumab, Rituximab, Robatumumab, Roledumab, Romosozumab, Rontalizumab, Rovelizumab, Ruplizumab, Sacituzumab, Samalizumab, Sarilumab, Satumomab, Secukimumab, Seribantumab, Setoxaximab, Sevirumab, Sibrotuzumab, Sifalimumab, Siltuximab, Siplizumab, Sirukumab, Sofituzumab, Solanezumab, Solitomab, Sonepcizumab, Sontuzumab, Stamulumab, Sulesomab, Suvizumab, Tabalumab, Tacatuzumab, Tadocizumab, Talizumab, Tanezumab, Taplitumomab, Tarextumab, Tefibazumab, Telimomab aritox, Tenatumomab, Teneliximab, Teplizumab, Tesidolumab, TGN 1412, Ticlimumab, Tildrakizumab, Tigatuzumab, TNX-650, Tocilizumab, Toralizumab, Tosatoxumab, Tositumomab, Tovetumab, Tralokimumab, Trastuzumab, TRBS07, Tregalizumab, Tremelimumab, Trevogrumab, Tucotuzumab, Tuvirumab, Ublituximab, Ulocuplumab, Urelumab, Urtoxazumab, Ustekimumab, Vandortuzumab, Vantictumab, Vanucizumab, Vapaliximab, Varlimumab, Vatelizumab, Vedolizumab, Veltuzumab, Vepalimomab, Vesencumab, Visilizumab, Volocixumab, Vorsetuzumab, Votumumab, Zalutumimab, Zanolimumab, Zatuximab, Ziralimumab, Ziv-Aflibercept, and Zolimomab;

- **Topoisomerase inhibitors,** such as doxorubicin, daunorubicin, dactinomycin, eniposide, epirubicin, etoposide, idarubicin, irinotecan, mitoxantrone, topotecan, and irinotecan;
- **Toxins,** such as Cholera toxin, ricin, *Pseudomonas* exotoxin, *Bordetella pertussis* adenylate cyclase toxin, diphtheria toxin, and caspase activators;

  **Kinase or VEGF inhibitors,** such as regorafenib (Stivarga®, Bayer); vandetanib (Caprelsa®, AstraZeneca); axitinib (Inlyta®, Pfizer); and lenvatinib (Lenvima®, Eisai); Raf inhibitors, such as sorafenib (Nexavar®, Bayer AG and Onyx); dabrafenib (Tafinlar®, Novartis); and vemurafenib (Zelboraf®, Genentech/Roche); MEK inhibitors, such as cobimetanib (Cotellic®, Exelexis/Genentech/Roche); trametinib (Mekinist®, Novartis); Bcr-Abl tyrosine kinase inhibitors, such as imatinib (Gleevec®, Novartis); nilotinib (Tasigna®, Novartis); dasatinib (Sprycel®, BristolMyersSquibb); bosutinib (Bosulif®, Pfizer); and ponatinib (Inclusig®, Ariad Pharmaceuticals); Her2 and EGFR inhibitors, such as gefitinib (Iressa®, AstraZeneca); erlotinib (Tarceeva®, Genentech/Roche/Astellas); lapatinib (Tykerb®, Novartis); afatinib (Gilotrif®, Boehringer Ingelheim); osimertinib (targeting activated EGFR, Tagrisso®, AstraZeneca); and brigatinib (Alunbrig®, Ariad Pharmaceuticals); c-Met and VEGFR2 inhibitors, such as cabozanitib (Cometriq®, Exelexis); and multikinase inhibitors, such as sunitinib (Sutent®, Pfizer); pazopanib (Votrient®, Novartis); ALK inhibitors, such as crizotinib (Xalkori®, Pfizer); ceritinib (Zykadia®, Novartis); and alectinib (Alecenza®, Genentech/Roche); Bruton's tyrosine kinase inhibitors, such as ibrutinib (Imbruvica®, Pharmacyclics/Janssen); and Flt3 receptor inhibitors, such as midostaurin (Rydapt®, Novartis), tivozanib (Aveo Pharmaecuticals); vatalanib (Bayer/Novartis); lucitanib (Clovis Oncology); dovitinib (TKI258, Novartis); Chiauanib (Chipscreen Biosciences); CEP-11981 (Cephalon); linifanib (Abbott Laboratories); neratinib (HKI-272, Puma Biotechnology); radotinib (Supect®, IY5511, Il-Yang Pharmaceuticals, S. Korea); ruxolitinib (Jakafi®, Incyte Corporation); PTC299 (PTC Therapeutics); CP-547,632 (Pfizer); foretinib (Exelexis, GlaxoSmithKline); quizartinib (Daiichi Sankyo) and motesanib (Amgen/Takeda);

In a non-limitative manner, ASDs of the invention may be combined, alone or in the form of a kit-of-parts, to one or more of the following anti-cancer drugs or compounds: ABVD, AC, ACE, Abiraterone (Zytiga®), Abraxane, Abstral, Actinomycin D, Actiq, Adriamycin, Afatinib (Giotrif®), Afinitor, Aflibercept (Zaltrap®), Aldara, Aldesleukin (IL-2, Proleukin or interleukin 2), Alemtuzumab (MabCampath), Alkeran, Amsacrine (Amsidine, m-AMSA), Amsidine, Anastrozole (Arimidex®), Ara C, Aredia, Arimidex, Aromasin, Arsenic trioxide (Trisenox®, ATO), Asparaginase (Crisantaspase®, Erwinase®), Axitinib (Inlyta®), Azacitidine (Vidaza®), BEACOPP, BEAM, Bendamustine (Levact®), Bevacizumab (Avastin), Bexarotene (Targretin®), Bicalutamide (Casodex®), Bleomycin, Bleomycin, etoposide and platinum (BEP), Bortezomib (Velcade®), Bosulif, Bosutinib (Bosulif), Brentuximab (Adcetris®), Brufen, Buserelin (Suprefact®), Busilvex, Busulfan (Myleran, Busilvex), CAPE-OX, CAPOX, CAV, CAVE, CCNU, CHOP, CMF, CMV, CVP, Cabazitaxel (Jevtana®), Cabozantinib (Cometriq®), Caelyx, Calpol, Campto, Capecitabine (Xeloda®), Caprelsa, Carbo MV, CarboTaxol, Carboplatin, Carboplatin and etoposide, Carboplatin and paclitaxel, Carmustine (BCNU, Gliadel®), Casodex, Ceritinib (Zykadia®), Cerubidin, Cetuximab (Erbitux®), ChlVPP, Chlorambucil (Leukeran®), Cisplatin, Cisplatin and Teysuno, Cisplatin and capecitabine (CX), Cisplatin, etoposide and ifosfamide (PEI), Cisplatin, fluorouracil (5-FU) and trastuzumab, Cladribine (Leustat®, LITAK), Clasteon, Clofarabine (Evoltra®), Co-codamol (Kapake®, Solpadol®, Tylex®), Cometriq, Cosmegen, Crisantaspase, Crizotinib (Xalkori®), Cyclophosphamide, Cyclophosphamide, thalidomide and dexamethasone (CTD), Cyprostat, Cyproterone acetate (Cyprostat®), Cytarabine (Ara C, cytosine arabinoside), Cytarabine into spinal fluid, Cytosine arabinoside, DHAP, DTIC, Dabrafenib (Tafinlar®), Dacarbazine (DTIC), Dacogen, Dactinomycin (actinomycin D, Cosmegen®), Dasatinib (Sprycel), Daunorubicin, De Gramont, Decapeptyl SR, Decitabine (Dacogen®), Degarelix (Firmagon®), Denosumab (Prolia®, Xgeva®), Depocyte, Dexamethasone, Diamorphine, Disodium pamidronate, Disprol, Docetaxel (Taxotere®), Docetaxel, cisplatin and fluorouracil (TPF), Doxifos, Doxil, Doxorubicin (Adriamycin), Doxorubicin and ifosfamide (Doxifos), Drogenil, Durogesic, EC, ECF, EOF, EOX, EP, ESHAP, Effentora, Efudix, Eldisine, Eloxatin, Enzalutamide, Epirubicin (Pharmorubicin®), Epirubicin cisplatin and capecitabine (ECX), Epirubicin, carboplatin and capecitabine (ECarboX), Eposin, Erbitux, Eribulin (Halaven®), Erlotinib (Tarceva®), Erwinase, Estracyt, Etopophos, Etoposide (Eposin®, Etopophos®, Vepesid®), Everolimus (Afinitor®), Evoltra, Exemestane (Aromasin®), FAD, FEC, FEC-T chemotherapy, FMD, FOLFIRINOX, FOLFOX, Faslodex, Femara, Fentanyl, Firmagon, Fludara, Fludarabine (Fludara®), Fludarabine, cyclophosphamide and rituximab (FCR), Fluorouracil (5FU), Flutamide, Folinic acid, fluorouracil and irinotecan (FOLFIRI), Fulvestrant (faslodex®), G-CSF, Gefitinib (Iressa), GemCarbo (gemcitabine and carboplatin), GemTaxol, Gemcitabine (Gemzar), Gemcitabine and capecitabine (GemCap), Gemcitabine and cisplatin (GC), Gemcitabine and paclitaxel (GemTaxol®),Gemzar,Giotrif, Gliadel, Glivec, Gonapeptyl, Depot, Goserelin (Zoladex®), Goserelin (Zoladex®, Novgos®), Granulocyte colony stimulating factor (G-CSF), Halaven,Herceptin, Hycamtin, Hydrea, Hydroxycarbamide (Hydrea®), Hydroxyurea, I-DEX, ICE, IL-2, IPE, Ibandronic acid, Ibritumomab (Zevalin®), Ibrutinib (Imbruvica®), Ibuprofen (Brufen®, Nurofen®), Iclusig, Idarubicin (Zavedos®), Idarubicin and dexamethasone, Idelalisib (Zydelig®), Ifosfamide (Mitoxana®), Imatinib (Glivec®), Imiquimod cream (Aldara®), Imnovid, Instanyl, Interferon (Intron A), Interleukin, Intron A, Ipilimumab (Yervoy®), Iressa, Irinotecan (Campto®), Irinotecan and capecitabine (Xeliri®), Irinotecan de Gramont, Irinotecan modified de Gramont, Javlor, Jevtana, Kadcyla, Kapake, Keytruda, Lanreotide (Somatuline®), Lanvis, Lapatinib (Tyverb®), Lenalidomide (Revlimid®), Letrozole (Femara®), Leukeran, Leuprorelin (Prostap®, Lutrate®), Leustat, Levact, Liposomal doxorubicin, Litak, Lomustine (CCNU), Lynparza, Lysodren, MIC, MMM, MPT, MST Continus, MVAC, MVP, MabCampath, Mabthera, Maxtrex, Medroxyprogesterone acetate (Provera), Megace, Megestrol acetate (Megace®), Melphalan (Alkeran®), Mepact, Mercaptopurine (Xaluprine®), Methotrexate (Maxtrex), Methyl prednisolone, Mifamurtide (Mepact®), Mitomycin C, Mitotane, Mitoxana, Mitoxantrone (Mitozantrone®), Morphgesic SR, Morphine, Myleran, Myocet, Nab-paclitaxel, Nab-paclitaxel (Abraxane®), Navelbine, Nelarabine (Atriance®), Nexavar, Nilotinib (Tasigna®), Nintedanib (Vargatef®), Nipent, Nivolumab (Opdivo®), Novgos, Nurofen, Obinutuzumab (Gazyvaro®), Octreotide, Ofatumumab (Arzerra®), Olaparib (Lynparza®), Oncovin, Onkotrone, Opdivo, Oramorph, Oxaliplatin (Eloxatin), Oxaliplatin and capecitabine (Xelox®), PAD, PC (paclitaxel and carboplatin, CarboTaxol), PCV, PE, PMitCEBO, POMB/ACE, Paclitaxel (Taxol®), Paclitaxel and carboplatin, Pamidronate, Panadol, Panitumumab (Vectibix®), Paracetamol, Pazopanib (Votrient®), Pembrolizumab (Keytruda), Pemetrexed (Alimta®), Pemetrexed and carboplatin, Pemetrexed and cisplatin, Pentostatin (Nipent®), Perjeta, Pertuzumab (Perjeta®), Pixantrone (Pixuvri®), Pixuvri, Pomalidomide (Imnovid®), Ponatinib, Potactasol, Prednisolone, Procarbazine, Proleukin, Prolia, Prostap, Provera, Purinethol, R-CHOP, R-CVP, R-DHAP, R-ESHAP, R-GCVP, RICE, Raloxifene, Raltitrexed (Tomudex®), Regorafenib (Stivarga®), Revlimid, Rituximab, (Mabthera®), Sevredol, Sodium clodronate (Bonefos®, Clasteon®, Loron®), Solpadol, Sorafenib (Nexavar®), Steroids (dexamethasone, prednisolone, methylprednisolone), Streptozocin (Zanosar®), Sunitinib (Sutent®), Sutent, TAC, TIP, Tafinlar, Tamoxifen, Tarceva, Targretin, Tasigna, Taxol, Taxotere, Taxotere and cyclophosphamide (TC), Temodal, Temozolomide, (Temodal®), Temsirolimus (Torisel®), Tepadina, Teysuno, Thalidomide, Thiotepa (Tepadina®), Tioguanine (thioguanine®, 6-TG, 6-tioguanine), Tomudex, Topotecan (Hycamtin, Potactasol), Torisel, Trabectedin (Yondelis), Trastuzumab (Herceptin®), Trastuzumab emtansine (Kadcyla®), Treosulfan, Tretinoin (Vesanoid®, ATRA), Triptorelin (Decapeptyl SR®, Gonapeptyl Depot®), Trisenox, Tylex, Tyverb,VIDE, Vandetanib (Caprelsa®), Vargatef, VeIP, Vectibix, Velbe, Velcade, Vemurafenib (Zelboraf®), Vepesid, Vesanoid, Vidaza, Vinblastine (Velbe®), Vincristine, Vincristine, actinomycin D (dactinomycin®) and cyclophosphamide (VAC), Vincristine, actinomycin and ifosfamide (VAI), Vincristine, doxorubicin and dexamethasone (VAD), Vindesine (Eldisine®), Vinflunine (Javlor®), Vinorelbine (Navelbine®),Vismodegib (Erivedge®), Votrient, XELOX, Xalkori, Xeloda, Xgeva, Xtandi, Yervoy, Yondelis, Z-DEX, Zaltrap, Zanosar, Zavedos, Zelboraf, Zevalin, Zoladex (breast cancer), Zoladex (prostate cancer), Zoledronic acid (Zometa®), Zometa, Zomorph, Zydelig, Zytiga.

According to a particular embodiment, an ASD, as described herein, can be combined with various chemotherapies, immunotherapy (e.g. check-point inhibitors, monoclonal antibodies), anti-tumoral vaccines, RNA vaccines, magnetic particles, intravascular microrobots, radiotherapy, surgery, ultrasounds or other anti-tumoral therapies.

Therefore, the present invention further provides an ASD as defined in the present invention or a pharmaceutical composition as defined in the present invention for use as an antitumor agent intended for patients who are also treated with anyone of immunotherapy, anti-tumoral vaccines, RNA vaccines, radiotherapy, surgery, ultrasounds or other anti-tumoral therapies.

According to one embodiment, the present invention relates to the ASD for use as defined above or the pharmaceutical composition for use as defined above, wherein the level of an ASD as defined above, in a blood, plasma, tissue, saliva, and/or serum sample of the patient is measured during the use.

According to another embodiment, the present invention also relates to the ASD for use as defined above or the pharmaceutical composition for use as defined above, wherein the use is intended for a patient whose level of an ASD as defined above, in a blood, plasma, tissue, saliva, and/or serum sample of the patient is measured during the use.

According to still another embodiment, the present invention also relates to the ASD for use as defined above or the pharmaceutical composition for use as defined above, wherein a presence and/or expression level of miR-124 in a blood and/or tissue sample of the patient, is measured prior to and/or during the use, in particular for monitoring efficacy of the use and/or response to the use.

Hereinafter, the present invention will be described in more detail with reference to the following examples. These examples are provided to illustrate the present invention and should not be construed as limiting the scope and spirit of the present invention.

**EXAMPLES**

In the present text, the symbol « ~ » means about.

MATERIALS

The materials and equipment used for the present disclosure are listed in Table 1 below.

**Table 1**

| Materials | | |
|---|---|---|
| **Description** | **Suppliers** | |
| Kollidon VA64 | BASF | |
| PVP K30 | BASF | |
| Acetonitrile (ACN) | VWR | |
| Trifluoroacetic acid (HPLC grade) | VWR | |
| Dichloromethane (DCM) | VWR | |
| Methanol (MeOH) | VWR | |
| Ethanol absolute (EtOH) | VWR | |
| **Equipment** | | |
| **Description** | **Model** | **Manufacturer** |
| HPLC | 1260 Infinity II | Agilent Technologies |
| Sonicator | Ultrasonic cleaner USC-TH | VWR |
| SEM | Tescan Vega 3 Scanning Electron Microscope | Tescan Bruno |
| XRPD | Bruker D2 Phaser | Karlsruhe |
| mDSC | Q1000 TA | TA Instruments |
| TGA | SDT Q600 | TA Instrument |
| PSD | Helos | Sympatec |

**Example 1: Preparation of ASD according to the invention.**

Two batches of amorphous solid dispersions were prepared by spray-drying using the 4M8 Trix spray dryer (ProCepT, Belgium) equipped with a bi-fluid nozzle. The list of parameters applied to spray dryer are summarized in table 2 below.

**Table 2**

| Parameter | Condition |
|---|---|
| Nozzle | 0.6 mm |
| Airspeed | 0.35 $m^3$/min |
| Nozzle air pressure | 2 Bar |
| Inlet temperature | 90 °C |
| Outlet temperature | 56-57 °C |
| Cyclone | Small |
| Flow rate | 3 mL/min |
| Nozzle flow rate | ~ 8 L/h |

Initially, 1.4 g of ABX464 was dissolved in 50 mL of MeOH followed by filtration through a 0.22 μm PVDF filtering membrane (Stericup Quick Release, Durapore). Afterwards 2.6 g of polymer (Kollidon® VA64 or Kollidon® K30 from BASF) were weighed and added to the previous ABX464 solution. The total solid content (ABX464 and polymer) in the spray drying solution was maintained at *ca.* 8% w/w. The mixture was kept under stirring until a homogenous solution was obtained. The solutions were fed to the spray dryer using a peristaltic pump at a flow rate 3 mL/min. The solutions were then spray dried. The spray-drying solutions used for the preparation of the feasibility batches were prepared at an ABX464:polymer ratio of 35:65 w/w.

Fine white powders were obtained after spray-drying. Approximately 80% yield were obtained for the produced SDDs (78.5% ABX464: Kollidon® VA64 (also named in the present text ABX464:VA64) and 80.9% ABX464: Kollidon® K30 (also named in the present text ABX464:K30)).

**Example 2: Characterization of ASD according to the invention.**

Example 2.1 : UV-HPLC (ultraviolet - High Performance Liquid Chromatography)

The UV-HPLC method used for the analysis of ABX464 is detailed in Table 3a below. For each HPLC experiment, two standard solutions (A and B) were prepared at 0.1 mg/mL in diluent (ACN:$H_2O$ (50:50)).

Approximately 2.5 mg of ABX464 (API) was accurately weighed into a 25 mL volumetric flask. The volume was adjusted with diluent and the standards were sonicated for 15 minutes at ambient temperature (room temperature), to ensure that the API had fully dissolved. The solution was then filtered through a 0.2 μm PTFE syringe filter (13 mm diameter) and transferred into amber glass vials in preparation for HPLC analysis. Two blank samples (diluent) were injected first to ensure that the baseline was acceptable and that no interfering peaks had eluted. This was followed by 5 injections of standard A and 2 injections of standard B.(system suitability testing (SST) results are provided in Table 3b below).

**Table 3a**

| Parameter | Condition |
|---|---|
| Column | Zorbax Eclipse Plus C18, Rapid resolution, 3.5 μm, 150x4.6mm<br>Serial number: U5UXC10978 |
| Flow rate | 1.0 mL |
| Temperature | Room temperature |
| Detection wavelength | 350 nm |
| Injection volume | 5 μL |
| Diluent | ACN:$H_2O$ (50:50) |
| Retention time | ~8.4 minutes |
| Run time | 20 minutes |
| Mobile Phase | Solvent A: 0.05% Trifluoroacetic acid in $H_2O$<br>Solvent B: 0.05% Trifluoroacetic acid in ACN |

| Gradient | | Time | % B |
|---|---|---|---|
| | | 0.00 | 5.0 |
| | | 1.40 | 5.0 |
| | | 5.40 | 90.0 |
| | | 14.9 | 90.0 |
| | | 15.0 | 5.0 |
| | | 20.0 | 5.0 |

Table 3b

| Standards Preparation | | | |
|---|---|---|---|
| Samples | Weight API (mg) | Volume (mL) | Concentration (mg/mL) |
| Standard A | 2.553 | 25.0 | 0.102 |
| Standard B | 2.647 | 25.0 | 0.106 |
| Repeatability precision | | | | |
| Samples | Retention time (min) | Peak area (mAU.s) | Mean Peak Area (mAU.s) | %RSD [Must be <2.0%] |
| Standard A (Repeatability only) | 8.36 | 889.7 | 886.6 | 0.67 |
| | 8.37 | 882.4 | | |
| | 8.36 | 894.9 | | |
| | 8.37 | 879.8 | | |
| | 8.37 | 886.4 | | |
| Standard B | 8.37 | 948.3 | 936.3 | 1.81 |
| | 8.38 | 924.3 | | |
| Standard agreement | | | | |
| Samples | Standard A | Standard B | Standard Agreement (Must be 1.0±0.02) |
| Standard Concentration (mg/mL) | 0.102 | 0.106 | 1.019 |
| Mean Peak Area | 886.6 | 936.3 | |
| PASS | | | |

Standard repeatability

The mean main peak area and % relative standard deviation (%RSD) was calculated for both standards to evaluate system precision. The %RSD of the main peak of API for standard solution A must be <2.0%.

Standard agreement

The standard agreement ratio of the response factors of standard solutions A and B must be between 0.98 and 1.02 and was calculated using the following equation:

$$\text{standard agreement} = \frac{\text{Area}_A}{\text{Area}_B} \times \frac{\text{Conc}_B}{\text{Conc}_A}$$

Where AreaA and Area$_B$ are the mean areas under the API peak in standard solutions A and B, respectively, and ConcA and Conc$_B$ are the concentrations of API in standard solutions A and B, respectively.

28

Results for UV-HPLC :

As expected, the UV-HPLC assay confirmed that both SDDs kept about 35% by weight drug load after spray-drying **(at t=0h)** (see Table 4 below) and consequently about 65% by weight of the pharmaceutically acceptable carrier (here povidone or copovidone) relative to the combined weight of ABX464 and the pharmaceutically acceptable carrier.

**Table 4**

| Assay (Time point) | ABX464:Kollidon® VA64 SDD | | ABX464:Kollidon® K30 SDD | |
|---|---|---|---|---|
| | % API in SDD | % peak purity | % API in SDD | % peak purity |
| 0 h | 34.97 | 100 | 34.30 | 100 |

Example 2.2 : X-Ray powder diffraction (XRPD) and Modulated differential scanning calorimetry (mDSC) analysis

XRPD

X-ray Powder Diffraction (XRPD) analysis on the feed API material and SDDs was carried out using a Bruker D8 Advance powder diffractometer equipped with a Lynx Eye detector. The sample (*ca.* 5 mg) was located at the centre of a silicon sample holder. The samples were scanned using a step size of 0.04° two theta (2θ) in the range of 2° to 40° 2θ. The data was processed using DIFFRAC[plus] EVA software and the detailed parameters are summarised in Table 5 below.

**Table 5**

| Parameter | Condition |
|---|---|
| Instrument | Bruker D8 Advance |
| Scan mode | Continuous |
| Source | Copper, K$\alpha$1, K$\alpha$2 |
| Wavelength | K$\alpha$1 : 1.540562 K$\alpha$2 : 1.54439 (I2/I1 = 0.5) |
| 2 Theta Range (Start/Stop) | 2-40° 2θ |
| Detector | Lynx Eye |
| Generator/voltage | 35 kV/40 mA |
| 2 Theta Step Size | 0.04° |
| Time/Step (Dwell) | 1 seconds |

mDSC analysis

Modulated differential scanning calorimetry (mDSC) was used to investigate the thermal behaviour of the feed API and SDDs using a Q200 calorimeter (TA Instruments, USA). An inert atmosphere was maintained in the chamber by purging nitrogen at 50 mL/min. Approximately 2-5 mg of the sample was weighed into hermetic aluminium pan, equilibrated at 0°C, and after an isotherm of 5 minutes, heated at 5°C/min up to 160 °C. A modulation period of 40 seconds with an amplitude temperature of 1 °C was applied. The data were processed using Universal Analysis 2000 software.

Results for XRPD and mDSC

Immediately post preparation (t=0h), both SDDs were confirmed amorphous by XRPD (figure 3) and mDSC (Figures 4 and 5) for physical form determination.

Figure 3 also comprises a XRPD diagram of ABX464 in its unique crystalline form in order to prove the non crystalline form of both SDDs.

Moreover, only one $T_g$ value which is different from the $T_g$ of single polymers and no ABX464 melting peak (present on ABX464 at about 120 °C) were observed in mDSC analysis of both SDDs. These observations suggested the formation of good homogeneous dispersions of the ABX464 in both polymer matrices (no signs of phase separation, that is to say presence of single $T_g$). However it is possible to observe a difference in the $T_g$ obtained for both SDDs (about 92 °C for ABX464:VA64 (figure 5) and about 135 °C for ABX464:K30 (figure 4).

Hence, these results demonstrate that ASD according to the invention are under amorphous form and also form a homogeneous dispersion of ABX464 in the polymer's matrix.

Example 2.3 : Scanning electron microscopy (SEM)

Particle shape and surface topography of the SDDs were examined by scanning electron microscopy (SEM). Approximately 1-2 mg of sample was mounted onto an aluminium stub using conductive double-sided carbon adhesive tape, sputter coated to 10 nm with gold in a Quorum Q150ES sputter coater (Quorum Technologies Ltd, UK) and photographed using a Tescan Vega3 scanning electron microscope (Tescan Bruno, Czech Republic). Magnification details and beam voltages are included with the scanning electron micrographs in this report.

Results for SEM

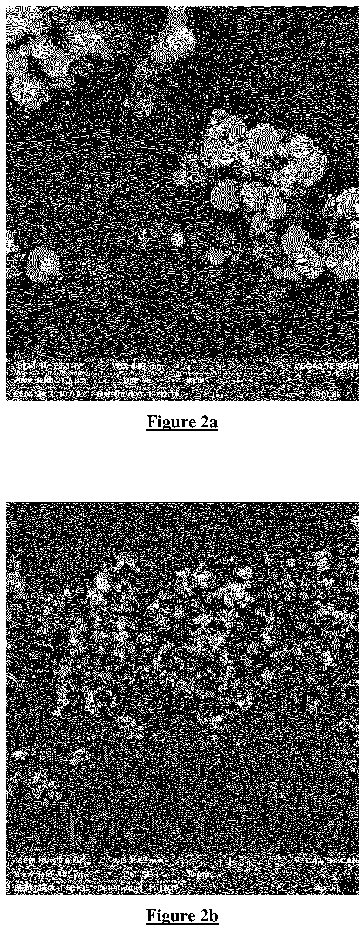

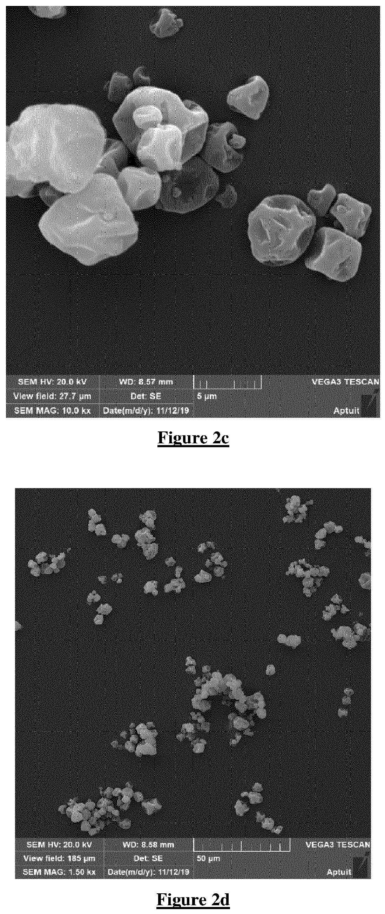

Immediately post preparation (t=0h), ABX464:VA64 and ABX464:K30 SDDs were evaluated by SEM in order to observe the particles' morphology after spray-drying process. Figures 2a, 2b, 2c and 2d show slightly different morphology for both SDDs. At the highest used magnification (10 kx, figures 2a and 2c) it is possible to observe that ABX464:VA64 SDD presents spherical particles whereas ABX464:K30 SDD consists of more irregular shaped particles. Concerning to particle size it is possible to observe that both SDDs have comparable size, although ABX464:VA64 particles appear to be slightly larger due to the observed higher agglomeration of these particles (when compared to ABX464:K30 SDD).

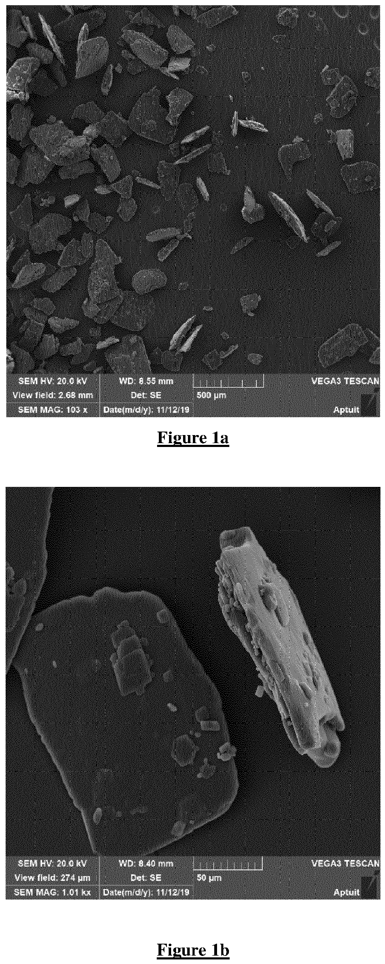

To compare with ABX464 in its unique crystalline form, the morphology of the particles were also assessed by SEM (see figures 1a and 1b). These figures show laminar shaped particles with large particle size ranging from 50-200 $\mu$m.

Example 2.4 : Thermogravimetric analysis (TGA)

Thermal gravimetric analysis was used to quantify the level of residual water/solvent. Simultaneous differential technique was used (SDT, Q500 TA Instrument) which is able to provide simultaneously TGA and DSC signals. Approximately 5-10 mg of material was weighed into alumina pan and loaded into the instrument held at room temperature and under nitrogen at a flow rate of 60mL/min. The sample was then heated to 350°C at a rate of 10°C/min and the sample weight recorded. The data were processed using Universal Analysis 2000 software.

Results for TGA

Immediately post preparation (t=0h), the SDDs were analysed by TGA for solvent loss determination.

The TGA thermograms (Figure 6) show a higher solvent loss in ABX464:K30 SDD (2.3%) when compared to ABX464:VA64 SDD (1.0%).

**Example 3: Two-step dissolution / precipitation Fasted and Fed Human *in-vitro* models**

ABX464 in its unique crystalline form and ABX464 :VA64 ASD (prepared according to example 1 above) were tested by using both Fasted and Fed Human *in-vitro* models (respectively named Fassif and Fessif models). These models simulate respectively fasted and fed state gastrointestinal fluids for dissolution testing.

For the Fasted in-vitro model, 11.4mg of ABX464:VA64 ASD (4.0mg equivalent ABX464) were weighed in 6 different vials. In all vials, 1ml of FaSSGF pH1.2 solution pre-heated at 37°C were added. Suspension were then vortexed at 37°C. After 15min vortex, suspension of the first vial was centrifuged 5min at 18000 rpm and filtered under 0.45$\mu$m filter (Millex LCR filter ref. SLCR0.13NK) for HPLC-UV dosage of supernatant. After 30min vortex, suspension of the second vial was centrifuged 5min at 18000 rpm and filtered under 0.45$\mu$m filter (Millex LCR filter ref. SLCR0.13NK) for HPLC-UV dosage of supernatant.

After 30min vortex, 1ml of FaSSIF x2 pH6.5 / Sodium bicarbonate 90/10 v/v pre-heated at 37°C was added on other 4 vials. Suspension were then vortexed at 37°C. After 15min vortex, suspension of the third vial was centrifuged 5min at 18000 rpm and filtered under 0.45$\mu$m filter (Millex LCR filter ref. SLCR0.13NK) for HPLC-UV dosage of supernatant. After 30min vortex, suspension of the fourth vial was centrifuged 5min at 18000 rpm and filtered under 0.45$\mu$m filter (Millex LCR filter ref. SLCR0.13NK) for HPLC-UV dosage of supernatant. After 60min vortex, suspension of the fifth vial was centrifuged 5min at 18000 rpm and filtered under 0.45$\mu$m filter (Millex LCR filter ref. SLCR0.13NK) for HPLC-UV dosage of supernatant. After 120min vortex, suspension of the sixth vial was centrifuged 5min at 18000 rpm and filtered under 0.45$\mu$m filter (Millex LCR filter ref. SLCR0.13NK) for HPLC-UV dosage of supernatant.

For the Fed in-vitro model, 11.4mg of ABX464:VA64 ASD (4.0mg equivalent ABX464) were weighed in 7 different vials. In all vials, 1ml of FeSSGF pH3.0 solution pre-heated at 37°C were added. Suspension were then vortexed at 37°C. After 15min vortex, suspension of the first vial was centrifuged 5min at 18000 rpm and filtered under 0.45μm filter (Millex LCR filter ref. SLCR0.13NK) for HPLC-UV dosage of supernatant. After 30min vortex, suspension of the second vial was centrifuged 5min at 18000 rpm and filtered under 0.45μm filter (Millex LCR filter ref. SLCR0.13NK) for HPLC-UV dosage of supernatant. After 60min vortex, suspension of the third vial was centrifuged 5min at 18000 rpm and filtered under 0.45μm filter (Millex LCR filter ref. SLCR0.13NK) for HPLC-UV dosage of supernatant.

After 60min vortex, 0.5ml of FeSSIF x3 pH5.0 pre-heated at 37°C was added on other 4 vials. Suspension were then vortexed at 37°C. After 15min vortex, suspension of the fourth vial was centrifuged 5min at 18000 rpm and filtered under 0.45μm filter (Millex LCR filter ref. SLCR0.13NK) for HPLC-UV dosage of supernatant. After 30min vortex, suspension of the fifth vial was centrifuged 5min at 18000 rpm and filtered under 0.45μm filter (Millex LCR filter ref. SLCR0.13NK) for HPLC-UV dosage of supernatant. After 60min vortex, suspension of the sixth vial was centrifuged 5min at 18000 rpm and filtered under 0.45μm filter (Millex LCR filter ref. SLCR0.13NK) for HPLC-UV dosage of supernatant. After 120min vortex, suspension of the seventh vial was centrifuged 5min at 18000 rpm and filtered under 0.45μm filter (Millex LCR filter ref. SLCR0.13NK) for HPLC-UV dosage of supernatant.

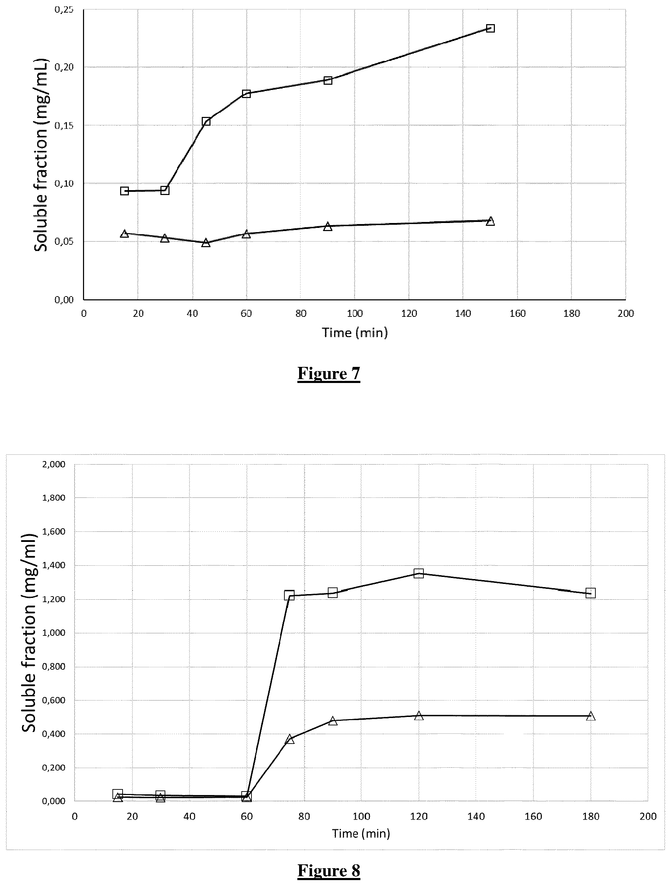

As shown in figures 7 and 8, ABX464 :VA64 ASD present a higher solubility than ABX464 in its unique crystalline form in both models.

Indeed, in the FASSIF model (final pH = 6.5), the results indicate 0.230 mg/ml *versus* 0.070 mg/ml for the last measurement point (30min in FaSSGF pH1.2 + 120min in FaSSIF x2 pH6.5 / Sodium bicarbonate 90/10 v/v) and in the FESSIF model (final pH 5.0), the results indicate 1.234mg/ml *versus* 0.508 mg/ml for the last measurement point (60min in FeSSGF pH3.0 + 120min in FeSSIF x3 pH5.0).

Figure 9 shows the amorphous nature of residual ABX464 : VA64 ASD in suspension at the end of both Fasted and Fed Human in-vitro models (at time 30min + 120min for Fasted model and at time 60min + 120min for Fed model respectively).

Fasted Gastric medium - FaSSGF pH=1.2

Table 6

| Product | Concentration | Preparation (500 mL) |
|---|---|---|
| NaCl | 34 mM | Weight 1.0 g of Sodium chloride (NaCl) in about 400 mL of deionised water. Magnetic stirring to dissolve NaCl. Adjust the pH to 1.2 with HCl. Make up to 500 mL with deionised water. |
| HCl | qs pH 1.2 | |
| Water | qs 1000 ml | |

Fasted Intestinal medium - FaSSIF pH 6.5

Composition / Preparation of FaSSIF x2 (medium concentrated 2 times to take into account the dilution in the Fasted model)

Table 7

| Product | Concentration | Preparation (500 mL) |
|---|---|---|
| Na taurocholate (TC) | 6 mM | 1) Preparation buffer: In about 0.45 L of deionised water dissolve: |
| Lecithin S100 (L) | 1.5 mM | - 2 x 0.21 = 0.42 g of Sodium hydroxide pellets (NaOH) |
| NaH$_2$PO$_4$, 2H$_2$O | 65,9 mM | - 2 x 1.98 = 3.95 g of NaH$_2$PO$_4$, H$_2$O |
| NaCl | 212 mM | - 2 x 3.1 = 6.19 g of Sodium chloride (NaCl) |
| NaOH 1N or HCl 1N | qs pH 6,5 | - Adjust the pH to 6.5 with NaOH 1N or HCl 1N. - Make up to volume (0.5 L) with purified water at room temperature. |

(continued)

| Product | Concentration | Preparation (500 mL) |
|---|---|---|
| Water | qs 500 ml | 2) Add SIF powder (from Biorelevant)Weigh 2 x 1.12 = 2.24 g of SIF Powder. Add 0.25L of buffer at room temperature. Stir until powder is completely dissolved. Make up to 0.5L. **Remarks** : Let stand for 2 hours, stability 48h at RT |

The mixture 90% FaSSIF X2 (pH 6.5) + 10% Sodium Bicarbonate 80 g/L was prepared in order to keep a pH equal to 6.5 in the intestinal compartment after dilution.

Fed Gastric medium - FeSSGF pH=3.0

**Table 8**

| Product | Concentration | Preparation (100 mL) |
|---|---|---|
| Na taurocholate (TC) | 0,75 mM | 1)Preparation buffer: In about 95 mL of deionised water dissolve : 68 mg of $NaH_2PO_4$ and 193 mg of NaCl. Adjust the pH to 3.0 with HCl 1N. Make up to 100 mL with purified water at room temperature. |
| Lecithin (L) | 0.12 mM | |
| $NaH_2PO_4$ | 4.35 mM | |
| NaCl | 33 mM | |
| HCl 1N | qs pH=3.0 | 2) Add Fessif-V2 powder (from Biorelevant):Weigh 74 mg ofFessif-V2, Add 50 mL of buffer and stir until powder is completely dissolved. Make up to 100 mL with buffer at room temperature. **Remark** : Let stand for 1 hour, stability 48h at RT |
| Water | qs 100 mL | |

Fed Intestinal medium - FeSSIF pH=5.0

**Table 9**

| Product | Concentration | Preparation (100 mL) |
|---|---|---|
| Na taurocholate (TC) | 45 mM | 1) Preparation buffer: In about 95 mL of deionised water dissolve: |
| Lecithin S100 (L) | 11.25 mM | - 3 x 0.404 g = 1.212 g of Sodium hydroxide pellets (NaOH) |
| NaCl | 609 mM | - 3 x 0.865 g = 2.595 g of Glacial acetic acid |
| Acetic Acid | 433 mM | - 3 x 1.187 g = 3.561 g of Sodium chloride (NaCl) |
| NaOH 1N or HCl 1N | qs pH 5.0 | - Adjust the pH to 5.0 with NaOH 1N or HCl 1N. - Make up to volume 100 mL with purified water at room temperature. |
| Water | qs 100 ml | 2) Add SIF powder (from Biorelevant): Weigh 3 x 1.120 = 3.36 g of SIF powder. Add 50 mL of buffer and stir until powder is completely dissolved. Make up to 100 mL with buffer at RT. **Remark** : Let stand for 1 hour, stability 48h atRT |

The Fed Intestinal medium - FeSSIF pH=5.0 was concentrated 3 times to take into account the dilution in the Fed model.

**Example 4: Chemical and Physical stability of ABX464:VA64 ASD and ABX464 :K30 ASD in accordance with the invention**

The Physical and Chemical stability of ABX464 : VA64 ASD and ABX464 : K30 ASD has been studied by XRPD and HPLC-UV after 2 weeks under following stressing conditions:

• 25°C / 60% Relative Humidity

• 40°C / 75% Relative Humidity

For stability studies, -100 mg of ABX464 : VA64 ASD formulation were placed in 2 glass jars hermetically closed. In one jar, a saturated NaCl solution was previously dispensed in the bottom of vial to generate a 60% Relative humidity. On the second jar, a saturated NaBr solution was previously dispensed in the bottom of vial to generate a 75% Relative humidity. The first jar was then placed for 2 weeks in an oven controlled at 25°C. The second jar was then placed for 2 weeks in an oven controlled at 40°C.

Results for ABX464 :VA64 ASD and ABX464 :K30 ASD

The results are summarized in table 10 below. The same observations were obtained for each of the two tested ASD.

**Table 10**

|  | 25°C/60%RH (RH : relative humidity) | 40°C/75%RH (RH : relative humidity) |
|---|---|---|
| **2 weeks Visual inspection : color** | white | white |
| **2 weeks Visual inspection : nature** | powder | powder |
| **2 weeks physical stability : nature of solid** | amorphous | amorphous |
| **2 weeks chemical stability : sum of degradant** | 0.0% | 0.0% |

Figure 10 reveals the amorphous nature of ABX464 : VA64 ASD formulation after 2 weeks at 25°C/60%RH and 40°C/75%RH respectively.
Figure 11 reveals the amorphous nature of ABX464 : K30 ASD formulation after 2 weeks at 25°C/60%RH and 40°C/75%RH respectively.
Hence, Table 10 that reports results of HPLC-UV analysis reveals the absence of chemical degradation of ABX464 : VA64 ASD formulation after 2 weeks at 25°C/60%RH and 40°C/75%RH respectively and of ABX464 : K30 ASD formulation after 2 weeks at 25°C/60%RH and 40°C/75%RH respectively.
These two results for each of the two ABX464 : VA64 ASD and ABX464 : K30 ASD show the physical and chemical stability of the ABX464 : VA64 formulation and of the ABX464 : K30 formulation after 2 weeks at 25°C/60%RH and 40°C/75%RH respectively.

**Example 5: Pharmaceutical compositions under the form of a capsule in accordance with the invention comprising a ABX464: COPOVIDONE ASD (ABX464: VA64 ASD) according to the invention or a ABX464: POVIDONE ASD (ABX464: K30 ASD) according to the invention**

The following capsule was prepared with the ingredients in the respective amounts as specified below in table 11.

**Table 11**

| Ingredients | Function | Amount (in mg) / unit |
|---|---|---|
| ABX464: COPOVIDONE ASD (35/65 w/w%) (also named in the present text ABX464: VA64 ASD) as prepared according to example 1 | Active ingredient | 20.00 equivalents ABX464 |

(continued)

| Ingredients | Function | Amount (in mg) / unit |
|---|---|---|
| MANNITOL | Filler | 120.00 |
| PREGELATINIZED STARCH | Binder | 10.00 |
| TALC | Glidant | 0.85 |
| ZINC STEARATE | Lubricant | 0.85 |
| White opaque hard gelatin capsule, size 1 sold by Capsugel Belgium NV (Body composition: 2% $TiO_2$ and qsp 100% gelatin (bovine and/or porcine origin) Cap composition: 2% $TiO_2$ and qsp 100% gelatin (bovine and/or porcine origin)). | Capsule shell | 1 unit |

The pharmaceutical compositions in accordance with the invention are useful in the treatment and / or prevention of inflammatory diseases such as Inflammatory Bowel Disease, Rheumatoid Arthritis, pulmonary arterial hypertension, NASH and Multiple Sclerosis, diseases caused by viruses and/or cancer or dysplasia.

**Claims**

1.  An amorphous solid dispersion comprising 8-chloro-N-(4-(trifluoromethoxy)phenyl)quinolin-2-amine or a pharmaceutically acceptable salt thereof and at least one pharmaceutically acceptable carrier.

2.  The amorphous solid dispersion according to claim 1, wherein the pharmaceutically acceptable carrier is selected from a polymer, a sugar, an acid, a surfactant, a cyclodextrin, pentaerythritol, pentaerythrityl tetraacetate, urea, urethane, hydroxy alkyl xanthins and mixtures thereof.

3.  The amorphous solid dispersion according to claim 1 or claim 2, wherein the pharmaceutically acceptable carrier is a polymer which is selected from homopolymers of N-vinyl lactams, copolymers of N-vinyl lactams, and mixtures thereof, particularly from povidone, copovidone, polyvinyl caprolactam - polyvinyl acetate - polyethylene glycol, and mixtures thereof, more particularly from povidone, copovidone, and mixtures thereof, and still more particularly is copovidone.

4.  The amorphous solid dispersion according to anyone of the preceding claims, wherein the amorphous solid dispersion is a glass solution forming a homogeneous one-phase system, and 8-chloro-N-(4-(trifluoromethoxy)phenyl)quinolin-2-amine or a pharmaceutically acceptable salt thereof is under an amorphous form.

5.  The amorphous solid dispersion according to anyone of the preceding claims, wherein the weight ratio of 8-chloro-N-(4-(trifluoromethoxy)phenyl)quinolin-2-amine or a pharmaceutically acceptable salt thereof and the pharmaceutically acceptable carrier(s) is in the range of from 1:20 to 1:0.5, particularly of from 1:10 to 1:1, more particularly of from 1:2 to 1:1.5.

6.  The amorphous solid dispersion according to anyone of the preceding claims, wherein 8-chloro-N-(4-(trifluoromethoxy)phenyl)quinolin-2-amine or a pharmaceutically acceptable salt thereof is in an amount of from 5 % to 70 by weight, particularly of from 30 % to 40% by weight, more particularly from 33 % to 37% by weight, relative to the combined weight of 8-chloro-N-(4-(trifluoromethoxy)phenyl)quinolin-2-amine or a pharmaceutically acceptable salt thereof and the pharmaceutically acceptable carrier(s).

7.  The amorphous solid dispersion according to anyone of the preceding claims, wherein said pharmaceutically acceptable carrier(s) is(are) in an amount of from 30 % to 95% by weight, particularly of from 60 % to 70% by weight, more particularly from 63 % to 67% by weight relative to the combined weight of 8-chloro-N-(4-(trifluoromethoxy)phenyl)quinolin-2-amine or a pharmaceutically acceptable salt thereof and the pharmaceutically acceptable carrier(s).

8.  The amorphous solid dispersion according to anyone of the preceding claims, wherein said pharmaceutically acceptable carrier is a combination of a polymer(s) as defined in any one of claims 2 and 3 and of acid(s) as defined in claim 2, particularly citric acid, succinic acid, malic acid, fumaric acid, tartaric acid or mixtures thereof.

9. Process for the preparation of the amorphous solid dispersion as defined in anyone of claims 1 to 8, comprising the following steps:

   a) Dissolving 8-chloro-N-(4-(trifluoromethoxy)phenyl)quinolin-2-amine or a pharmaceutically acceptable salt thereof in a suitable solvent or mixture of solvents so as to obtain a solution;
   b) Adding to the thus obtained solution of step a) at least one pharmaceutically acceptable carrier as defined in the present invention;
   c) Optionally mixing the mixture obtained in step b); and
   d) Evaporating the solvent(s) to provide the amorphous solid dispersion.

10. The process according to claim 9, wherein the suitable solvent of step a) is any volatile solvent which is able to dissolve both the pharmaceutically acceptable carrier and ABX464 or a salt thereof, particularly the suitable solvent is selected from a $C_1$-$C_6$ alcohol, dichloromethane, acetonitrile, acetone, THF (tetrahydrofuran), diethyl ether and mixtures thereof, more particularly is selected from a $C_1$-$C_4$ monoalcohol, dichloromethane and mixtures thereof, still more particularly is selected from methanol, ethanol, dichloromethane, and mixtures thereof, even more particularly is methanol.

11. The process according to claim 9 or claim 10, wherein the evaporation step d) is carried out by spray - drying, holt-melt extrusion, or solvent evaporation method, particularly by spray - drying or holt-melt extrusion, more particularly by spray - drying.

12. Pharmaceutical composition comprising the amorphous solid dispersion as defined in anyone of claims 1 to 8, and at least one pharmaceutically acceptable excipient.

13. Pharmaceutical composition according to claim 12, wherein the pharmaceutical composition comprises 8-chloro-N-(4-(trifluoromethoxy)phenyl)quinolin-2-amine or a pharmaceutically acceptable salt thereof as the sole pharmaceutically active ingredient.

14. Process for the preparation of the pharmaceutical composition as defined in claim 12 or claim 13, comprising the following steps:

   a) Dissolving 8-chloro-N-(4-(trifluoromethoxy)phenyl)quinolin-2-amine or a pharmaceutically acceptable salt thereof in a suitable solvent or mixture of solvents so as to obtain a solution;
   b) Adding to the thus obtained solution of step a) at least one pharmaceutically acceptable carrier as defined in the present invention;
   c) Optionally mixing the mixture obtained in step b);
   d) Evaporating the solvent(s) to provide the amorphous solid dispersion,
   e) Mixing together the amorphous solid dispersion of step d) with excipient(s) to obtain the pharmaceutical composition; and
   f) Optionally coating the thus obtained pharmaceutical composition when a coated pharmaceutical composition is needed.

15. The amorphous solid dispersion as defined in anyone of the claims 1 to 8 or the pharmaceutical composition as defined in claim 12 or claim 13 for use as a medicament.

16. The amorphous solid dispersion as defined in anyone of the claims 1 to 8 or the pharmaceutical composition as defined in claim 12 or claim 13 for use in the treatment and/or prevention of an inflammatory disease.

17. The amorphous solid dispersion as defined in anyone of the claims 1 to 8 or the pharmaceutical composition as defined in claim 12 or claim 13 for use in the treatment and/or prevention of cancer.

18. The amorphous solid dispersion or the pharmaceutical composition for use according to anyone of claims 15 to 17, wherein a presence and/or expression level of miR-124 in a blood and/or tissue sample of the patient, is measured prior to and/or during the use, in particular for monitoring efficacy of the use and/or response to the use.

19. The amorphous solid dispersion as defined in anyone of the claims 1 to 8 or the pharmaceutical composition as defined in claim 12 or claim 13, for use in the treatment and/or prevention of diseases caused by viruses, in particular by retroviruses and more particularly by HIV, and more particularly for use for lowering viral load in a patient infected by a virus, in particular HIV, or a virus-related condition, with a long-lasting effect and absence of resistance.

20. The amorphous solid dispersion or the pharmaceutical composition for use according to anyone of claims 15 to 19, wherein the level of an ASD as defined in anyone of claims 1 to 8, in a blood, plasma, tissue, saliva, and/or serum sample of the patient is measured during the use.

SEM HV: 20.0 kV | WD: 8.55 mm | | VEGA3 TESCAN
View field: 2.68 mm | Det: SE | 500 µm |
SEM MAG: 103 x | Date(m/d/y): 11/12/19 | | Aptuit

**Figure 1a**

SEM HV: 20.0 kV | WD: 8.40 mm | | VEGA3 TESCAN
View field: 274 µm | Det: SE | 50 µm |
SEM MAG: 1.01 kx | Date(m/d/y): 11/12/19 | | Aptuit

**Figure 1b**

**Figure 2a**

**Figure 2b**

**Figure 2c**

**Figure 2d**

**Figure 3**

**Figure 4**

**Figure 5**

**Figure 6**

**Figure 7**

**Figure 8**

**Figure 9**

EP 3 858 336 A1

**Figure 10**

**Figure 11**

Europäisches
Patentamt

European
Patent Office

Office européen
des brevets

**EUROPEAN SEARCH REPORT**

Application Number

EP 20 30 5089

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2010/143169 A2 (SPLICOS SOC [FR]; CENTRE NAT RECH SCIENT [FR] ET AL.) 16 December 2010 (2010-12-16) * page 28, line 19 * * claim 9 * ----- | 1-20 | INV. A61K9/16 |

TECHNICAL FIELDS
SEARCHED (IPC)

A61K

The present search report has been drawn up for all claims

| Place of search | Date of completion of the search | Examiner |
|---|---|---|
| The Hague | 10 July 2020 | Muller, Sophie |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

........................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 20 30 5089

This annex lists the patent family members relating to the patent documents cited in the above-mentioned European search report.
The members are as contained in the European Patent Office EDP file on
The European Patent Office is in no way liable for these particulars which are merely given for the purpose of information.

10-07-2020

| Patent document cited in search report | Publication date | | Patent family member(s) | | Publication date |
|---|---|---|---|---|---|
| WO 2010143169 A2 | 16-12-2010 | AU | 2010258214 | A1 | 19-01-2012 |
| | | AU | 2010258294 | A1 | 19-01-2012 |
| | | AU | 2010258295 | A1 | 19-01-2012 |
| | | BR | PI1010768 | A2 | 01-11-2016 |
| | | BR | PI1010772 | A2 | 01-11-2016 |
| | | BR | PI1012892 | A2 | 13-03-2018 |
| | | BR | 122019013686 | B1 | 17-03-2020 |
| | | BR | 122019013687 | B1 | 14-04-2020 |
| | | CA | 2764024 | A1 | 16-12-2010 |
| | | CA | 2764026 | A1 | 16-12-2010 |
| | | CA | 2764027 | A1 | 16-12-2010 |
| | | CA | 2965791 | A1 | 16-12-2010 |
| | | CA | 3070823 | A1 | 16-12-2010 |
| | | CN | 102574835 | A | 11-07-2012 |
| | | CN | 102596935 | A | 18-07-2012 |
| | | CN | 102625804 | A | 01-08-2012 |
| | | CN | 103948594 | A | 30-07-2014 |
| | | CN | 104844510 | A | 19-08-2015 |
| | | CN | 104844570 | A | 19-08-2015 |
| | | CN | 104945378 | A | 30-09-2015 |
| | | CN | 104945379 | A | 30-09-2015 |
| | | CN | 106905232 | A | 30-06-2017 |
| | | CN | 106928194 | A | 07-07-2017 |
| | | CN | 106928205 | A | 07-07-2017 |
| | | CN | 109776496 | A | 21-05-2019 |
| | | CU | 20110228 | A7 | 15-04-2012 |
| | | CU | 20110229 | A7 | 21-06-2012 |
| | | CU | 20110230 | A7 | 15-04-2012 |
| | | CU | 20150024 | A7 | 29-09-2015 |
| | | DK | 2440545 | T3 | 22-07-2019 |
| | | EP | 2440545 | A2 | 18-04-2012 |
| | | EP | 2440546 | A2 | 18-04-2012 |
| | | EP | 2440547 | A2 | 18-04-2012 |
| | | EP | 3517534 | A1 | 31-07-2019 |
| | | EP | 3521283 | A1 | 07-08-2019 |
| | | ES | 2736198 | T3 | 26-12-2019 |
| | | HK | 1173153 | A1 | 10-10-2014 |
| | | HK | 1173447 | A1 | 21-07-2017 |
| | | HK | 1173726 | A1 | 28-10-2016 |
| | | HK | 1200363 | A1 | 07-08-2015 |
| | | HK | 1212702 | A1 | 17-06-2016 |
| | | HK | 1212990 | A1 | 24-06-2016 |
| | | HK | 1213246 | A1 | 30-06-2016 |
| | | HK | 1213247 | A1 | 30-06-2016 |
| | | HU | E045548 | T2 | 30-12-2019 |
| | | JP | 5826745 | B2 | 02-12-2015 |

For more details about this annex : see Official Journal of the European Patent Office, No. 12/82

EPO FORM P0459

page 1 of 3

## ANNEX TO THE EUROPEAN SEARCH REPORT
## ON EUROPEAN PATENT APPLICATION NO.

EP 20 30 5089

This annex lists the patent family members relating to the patent documents cited in the above-mentioned European search report.
The members are as contained in the European Patent Office EDP file on
The European Patent Office is in no way liable for these particulars which are merely given for the purpose of information.

10-07-2020

| Patent document cited in search report | Publication date | Patent family member(s) | Publication date |
|---|---|---|---|
| | | JP 5905385 B2 | 20-04-2016 |
| | | JP 6041671 B2 | 14-12-2016 |
| | | JP 6158250 B2 | 05-07-2017 |
| | | JP 6158251 B2 | 05-07-2017 |
| | | JP 2012529493 A | 22-11-2012 |
| | | JP 2012529494 A | 22-11-2012 |
| | | JP 2012529495 A | 22-11-2012 |
| | | JP 2015155453 A | 27-08-2015 |
| | | JP 2015187150 A | 29-10-2015 |
| | | JP 2015187151 A | 29-10-2015 |
| | | JP 2015187152 A | 29-10-2015 |
| | | KR 20120049860 A | 17-05-2012 |
| | | KR 20120051643 A | 22-05-2012 |
| | | KR 20120054585 A | 30-05-2012 |
| | | KR 20170092720 A | 11-08-2017 |
| | | KR 20170124646 A | 10-11-2017 |
| | | KR 20180100735 A | 11-09-2018 |
| | | KR 20180100736 A | 11-09-2018 |
| | | KR 20180100737 A | 11-09-2018 |
| | | KR 20180101727 A | 13-09-2018 |
| | | KR 20190018567 A | 22-02-2019 |
| | | KR 20190018568 A | 22-02-2019 |
| | | KR 20190018569 A | 22-02-2019 |
| | | KR 20190018570 A | 22-02-2019 |
| | | KR 20190018571 A | 22-02-2019 |
| | | MX 338823 B | 03-05-2016 |
| | | MX 340095 B | 27-06-2016 |
| | | MX 353776 B | 29-01-2018 |
| | | MX 359575 B | 03-10-2018 |
| | | MX 364279 B | 22-04-2019 |
| | | MX 364280 B | 22-04-2019 |
| | | MX 364282 B | 22-04-2019 |
| | | MX 364989 B | 17-05-2019 |
| | | MX 367601 B | 28-08-2019 |
| | | PL 2440545 T3 | 29-11-2019 |
| | | PT 2440545 T | 19-07-2019 |
| | | RU 2011149571 A | 20-07-2013 |
| | | RU 2011149572 A | 20-07-2013 |
| | | TR 201910781 T4 | 21-08-2019 |
| | | US 2012277230 A1 | 01-11-2012 |
| | | US 2012283265 A1 | 08-11-2012 |
| | | US 2012329796 A1 | 27-12-2012 |
| | | US 2014080831 A1 | 20-03-2014 |
| | | US 2014288120 A1 | 25-09-2014 |
| | | US 2015299129 A1 | 22-10-2015 |
| | | US 2015307478 A1 | 29-10-2015 |

EPO FORM P0459

For more details about this annex : see Official Journal of the European Patent Office, No. 12/82

page 2 of 3

**ANNEX TO THE EUROPEAN SEARCH REPORT
ON EUROPEAN PATENT APPLICATION NO.**

EP 20 30 5089

This annex lists the patent family members relating to the patent documents cited in the above-mentioned European search report.
The members are as contained in the European Patent Office EDP file on
The European Patent Office is in no way liable for these particulars which are merely given for the purpose of information.

10-07-2020

| Patent document cited in search report | Publication date | Patent family member(s) | Publication date |
|---|---|---|---|
| | | WO 2010143168 A2 | 16-12-2010 |
| | | WO 2010143169 A2 | 16-12-2010 |
| | | WO 2010143170 A2 | 16-12-2010 |
| | | ZA 201109031 B | 27-02-2013 |
| | | ZA 201109032 B | 27-02-2013 |

EPO FORM P0459

For more details about this annex : see Official Journal of the European Patent Office, No. 12/82

page 3 of 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010143169 A

**Non-patent literature cited in the description**

- **S. M. BERGE et al.** describe pharmaceutically acceptable salts in detail. *J. Pharmaceutical Sciences,* 1977, vol. 66, 1-19

- **PRASHANT et al.** Amorphous solid dispersion: a promising technique for improving oral bioavailability of poorly water-soluble drugs. *S. Afr. Pharm. J.,* 2018, vol. 85 (1), 50-56